# EUROPEAN PATENT APPLICATION

(11) **EP 4 650 445 A1**
(43) Date of publication of application: **19.11.2025**
(21) Application number: 23916298.5
(22) Date of filing: 27.12.2023
(51) Int. Cl.: C12N 15/11, C12Q 1/6869

(54) **INTERNAL STANDARD NUCLEIC ACID FOR GENOMIC OR METAGENOMIC ANALYSIS**

(30) Priority: 13.01.2023 JP 2023003917
(71) Applicant: National Institute of Advanced Industrial Science and Technology, Chiyoda-ku Tokyo 100-8921 (JP)
(72) Inventor: SEKIGUCHI, Yuji, Tsukuba-shi, Ibaraki 305-8560 (JP); TOURLOUSSE, Dieter, Tsukuba-shi, Ibaraki 305-8560 (JP); OHASHI, Akiko, Tsukuba-shi, Ibaraki 305-8560 (JP)
(74) Representative: HGF
(86) International application number: PCT/JP2023/046978
(87) International publication number: WO 2024/150685

(57) **Abstract**

Provided is a nucleic acid molecule comprising an artificial nucleic acid sequence and/or a complementary sequence thereof, or a partial fragment sequence thereof, the artificial nucleic acid sequence consisting of: (1) one copy each of artificial genes encoding the following non-naturally occurring sequences (a) to (p); (2) artificial intergenic sequences for linking the artificial genes, each independently consisting of a non-naturally occurring random sequence of 10 to 60 nucleotides in length; and (3) a front spacer sequence and an end spacer sequence, each independently consisting of a non-naturally occurring random sequence of 200 to 400 nucleotides in length.

## Description

### [Technical Field]

The present invention relates to internal standard nucleic acids for genomic or metagenomic analysis.

### [Background Art]

A wide variety of microorganisms inhabit all kinds of environments, including natural environments such as soil and oceans, animal intestines, and human habitations such as houses. In many cases, they are colonized by unique compositions in each environment, and such a community of microorganisms is called a microbiota. Microbiota have been analyzed using 16S rRNA genes by next-generation sequencing (NGS) or whole-genome shotgun metagenomics. In 16S rRNA gene analysis, PCR products amplified from 16S rRNA genes in the microbiota are comprehensively sequenced. In contrast, in whole-genome shotgun metagenomic analysis, whole genome DNA in the microbiota are comprehensively sequenced, allowing comprehensive analysis of the functional genes present in the microbiota and elucidating the functions possessed by the entire microbiota.

Whole-genome shotgun metagenomic analysis involves steps of extracting whole-genome DNA from a microbiota, randomly fragmenting the whole-genome DNA, sequencing the fragments, assembling the resulting fragment sequences (sequence reads) into a contiguous sequence (contig), and mapping the reads to the genome sequence deduced by the assembly, thereby quantifying the relative abundance of genes in the microbiota. However, the quantitative results are only a relative measure and do not provide an estimate of the absolute abundance of the detected microbial communities or functional genes. In addition, the above steps are subject to technical biases, and these biases must be accurately identified and adjusted for in order to obtain accurate results.

For absolute quantification and accuracy control, methods of correcting the measured value using an exogenous nucleic acid with a sequence not present in the sample (spike-in control) as an internal standard are known, and standard nucleic acids including artificial nucleic acid sequences not present in nature have been developed (Patent Document 1, Non-Patent Document 1). However, bioinformatics tools for assessing assembly quality, such as CheckM (Parks, et al., Genome Research, 2015, 25(7): 1043-55), typically provide accurate estimates of genome completeness and contamination level based on the presence or absence of specific single-copy marker genes within the assembled contigs, and thus cannot assess the quality of standard nucleic acid assemblies that do not contain such gene sequences as noted above. In addition, GC content is known to cause variation in sequencing coverage and reduce assembly accuracy (GC bias), and a standard nucleic acid for rigorous assessment of GC bias is also desired.

### [Prior Art]

### [Patent Document]

Patent Document 1: WO 2017/165864

### [Non-patent Document]

Non-Patent Document 1: Hardwick, et al., 2018, Nature Communications, Vol. 9, Article No: 3096

### [Summary of Invention]

### [Problem to be Solved by the Invention]

The present invention has been made for the purpose of providing internal standard nucleic acids for evaluating assembly quality in genomic or metagenomic analysis.

### [Means for Solving the Problems]

The present inventors have intensively researched, and as a result, succeeded in producing artificial nucleic acids capable of accurately evaluating the quality of assemblies.

That is, according to one embodiment, the present invention provides a nucleic acid molecule comprising an artificial nucleic acid sequence and/or a complementary sequence thereof, or a partial fragment sequence thereof, the artificial nucleic acid sequence consisting of: (1) one copy each of artificial genes encoding the following non-naturally occurring sequences (a) to (p): (a) an amino acid sequence of SEQ ID NO: 1 and a stop codon; (b) an amino acid sequence of SEQ ID NO: 2 and a stop codon; (c) an amino acid sequence of SEQ ID NO: 3 and a stop codon; (d) an amino acid sequence of SEQ ID NO: 4 and a stop codon; (e) an amino acid sequence of SEQ ID NO: 5 and a stop codon; (f) an amino acid sequence of SEQ ID NO: 6 and a stop codon; (g) an amino acid sequence of SEQ ID NO: 7 and a stop codon; (h) an amino acid sequence of SEQ ID NO: 8 and a stop codon; (i) an amino acid sequence of SEQ ID NO: 9 and a stop codon; (j) an amino acid sequence of SEQ ID NO: 10 and a stop codon; (k) an amino acid sequence of SEQ ID NO: 11 and a stop codon; (1) an amino acid sequence of SEQ ID NO: 12 and a stop codon; (m) an amino acid sequence of SEQ ID NO: 13 and a stop codon; (n) an amino acid sequence of SEQ ID NO: 14 and a stop codon; (o) an amino acid sequence of SEQ ID NO: 15 and a stop codon; and (p) an amino acid sequence of SEQ ID NO: 16 and a stop codon; (2) artificial intergenic sequences for linking the artificial genes, each independently consisting of a non-naturally occurring random sequence of 10 to 60 nucleotides in length; and (3) a front spacer sequence and an end spacer sequence, each independently consisting of a non-naturally occurring random sequence of 200 to 400 nucleotides in length.

The GC content of the artificial nucleic acid sequence is preferably 30 to 60%.

The artificial nucleic acid sequence is preferably selected from the group consisting of SEQ ID NOs: 17 to 22.

The partial fragment sequence is preferably at least 300 nucleotides in length.

According to one embodiment, the present invention also provides a nucleic acid molecule comprising an artificial nucleic acid sequence of SEQ ID NO: 23 and/or the complementary sequence thereof, or a partial fragment sequence thereof.

The partial fragment sequence is preferably at least 300 nucleotides in length.

### [Effects of the Invention]

According to one embodiment, the nucleic acid molecule of the present invention is composed of non-naturally occurring artificial nucleic acid sequences, while having artificial genetic sequences that are recognizable by tools such as CheckM. Therefore, the nucleic acid molecule of the present invention allows for the evaluation of assembly quality based on the presence or absence of single copy-marker genes, which is now commonly employed.

Also, according to one embodiment, the nucleic acid molecule of the present invention has an artificial nucleic acid sequence with strictly controlled GC content. Therefore, the nucleic acid molecules of the present invention permits a rigorous evaluation of the effect of GC bias on the assembly.

Also, the nucleic acid molecules of the present invention enable absolute quantification of genes present in microbiota.

### [Brief Description of Drawings]

[FIG. 1] FIG. 1 is a schematic drawing showing a procedure for generating an artificial nucleic acid sequence including an artificial coding sequence (CDS), using seqHMM3501 as an example.
[FIG. 2A] FIG. 2A is a drawing showing the layout of the 16 artificial CDSs in seqHMM3501, seqHMM5001, seqHMM5002, seqHMM5003, seqHMM6001 and seqHMM04.
[FIG. 2B] FIG. 2B is a drawing showing the GC content in seqHMM3501, seqHMM5001, seqHMM5002, seqHMM5003, seqHMM6001 and seqHMM04.
[FIG. 2C] FIG. 2C is a drawing showing the GC content in seqRANDOM01.
[FIG. 2D] FIG. 2D is a drawing showing the pairwise sequence identity of seqHMM5002 and seqHMM5003.
[FIG. 3A] FIG. 3A is a drawing showing the relationship between the coverage depth and the percentage of artificial nucleic acid sequences recovered by the assembly when seqHMM3501, seqHMM5001, seqHMM5002, seqHMM5003, seqHMM6001, seqHMM04 and seqRANDOM01 are analyzed individually.
[FIG. 3B] FIG. 3B is a drawing showing the relationship between the number of marker genes detected in the artificial nucleic acid sequences recovered by the assembly and the depth of coverage when seqHMM3501, seqHMM5001, seqHMM5002, seqHMM5003, seqHMM6001, seqHMM04, and seqRANDOM01 are analyzed individually.
[FIG. 4] FIG. 4 is a drawing showing the assembly completeness when an equimolar mixture of seqHMM3501, seqHMM5001, seqHMM5002, seqHMM5003, seqHMM6001, seqHMM04, and seqRANDOM01 is analyzed.
[FIG. 5A] FIG. 5A is a plot of the relative coverage and GC content along the positions in seqHMM04 and seqRANDOM01.
[Fig. 5B] FIG. 5B is a scatter plot of the relationship between the relative coverage and GC content along the positions in seqHMM04 and seqRANDOM01.
[FIG. 6] FIG. 6 is a plot of the abundance of each artificial nucleic acid (measured vs. estimated) in two types of mixtures containing seqHMM3501, seqHMM5001, seqHMM5002, seqHMM5003, seqHMM6001, seqHMM04, and seqRANDOM01 in different proportions.
[FIG. 7] FIG. 7 is a plot of the relative ratio (measured vs. estimated) of the artificial nucleic acids added to a human fecal microbiota DNA sample compared to human fecal microbiota DNA.

### [Description of Embodiments]

The present invention is described in detail below, but it is not limited to the embodiments described herein.

According to the first embodiment, the present invention is a nucleic acid molecule comprising an artificial nucleic acid sequence and/or a complementary sequence thereof, or a partial fragment sequence thereof, the artificial nucleic acid sequence consisting of: (1) one copy each of artificial genes encoding the following non-naturally occurring sequences (a) to (p): (a) an amino acid sequence of SEQ ID NO: 1 and a stop codon; (b) an amino acid sequence of SEQ ID NO: 2 and a stop codon; (c) an amino acid sequence of SEQ ID NO: 3 and a stop codon; (d) an amino acid sequence of SEQ ID NO: 4 and a stop codon; (e) an amino acid sequence of SEQ ID NO: 5 and a stop codon; (f) an amino acid sequence of SEQ ID NO: 6 and a stop codon; (g) an amino acid sequence of SEQ ID NO: 7 and a stop codon; (h) an amino acid sequence of SEQ ID NO: 8 and a stop codon; (i) an amino acid sequence of SEQ ID NO: 9 and a stop codon; (j) an amino acid sequence of SEQ ID NO: 10 and a stop codon; (k) an amino acid sequence of SEQ ID NO: 11 and a stop codon; (1) an amino acid sequence of SEQ ID NO: 12 and a stop codon; (m) an amino acid sequence of SEQ ID NO: 13 and a stop codon; (n) an amino acid sequence of SEQ ID NO: 14 and a stop codon; (o) an amino acid sequence of SEQ ID NO: 15 and a stop codon; and (p) an amino acid sequence of SEQ ID NO: 16 and a stop codon; (2) artificial intergenic sequences for linking the artificial genes, each independently consisting of a non-naturally occurring random sequence of 10 to 60 nucleotides in length; and (3) a front spacer sequence and an end spacer sequence, each independently consisting of a non-naturally occurring random sequence of 200 to 400 nucleotides in length.

The artificial nucleic acid sequence in the nucleic acid molecule of the present embodiment comprises, as components (1), one copy each of the artificial genes encoding the following sequences (a) to (p). In the sequence, X represents any amino acid residue.
(a)MXXKIKXGDXVXVIXGKXKGXXGXVXXVXXXXXXVIVEGVXXXKKXX KXXXXXXXXGXXXXXEXPIXXSNVXXXXXXXXXXXXVXXRXXXXXXKXRXXXX XGXXI (SEQ ID NO: 1) and a stop codon
(b)MXXXIXXLXXXXXXXXXXXXFXXGXXVXVXXXIXEGXXXRXQXFXGX VIXXXXXGXXXXXXVXKXXXGXGVERXFXXXXXXIXXIXVXXXGXVXRAXLXYL RXXXGKXXKIKXXX (SEQ ID NO: 2) and a stop codon
(c)MMAXXXRXXRVXXXIXXXIXXXLXXXIXDXXXXXXXVXXVEXSXDLXX XXVFVXXLXDXXXXXXXVXXLXXAXGFIXXXLXXXXXLXXXPXLXFXXDXSLXX XXRIXXLIXXLXXX (SEQ ID NO: 3) and a stop codon
(d)MXXXFXXXPLXXGXGXTLGXXLRRVLLXXIXGXAIXXXXIXXXXXEFXX XXGVXEDVXXIIXNLKXLXXXXXXXXXXXXXXXXXXXXXXXXAXXXXXXXXXVE VXXXXXXIXXLXXXXXLXIXLXVXXGXGYXXXXXXXXXXXXXXXIXVDAXFXPV XXVXYXVXXXXXXXXXXXDXLXLXIXTXXXXXXXXALXXAXXXLXXXLXXXXX XXXXXXXXXXXXXXXXXXXXXXXXXXXXIXXLDLSXRXXNCLXXXXIXXLXELV XXXXXXLXXXXNLGXKSXXEIXXXLXXXXLXLXXX (SEQ ID NO: 4) and a stop codon
(e)MFXDXXXXXVXXGXGGXGXXXXXXEXYXXXGGPXGGXGGXGGXVXX XXXXXXXXLXXXXXXXXXXAXXGXXGXXXXXXGXXXXXXXIXVPXGXXVXXXX XXXXXXXXXXXXXXXXXXXGGXXGXGNXXFXXXXXXXPXXXXXGXXXXXXXL XLXLXXLADVGLVGXXXXGKSXLLXXXXXXXXXIXXYXFXTXXPXLGXXXXXXX XXXXXADIPGLIXXXXXGXGLGXXFLXHIXXXXXLXXLIXXXXXXXXXXXXXXXX XXXXLXXYXXXLXXXXXXXXXXKXDXXXXXXXXXXXXXXXXXXXXXXXXXXX XXXXXXXXXXXXXXXXXX (SEQ ID NO: 5) and a stop codon
(f)MXXVAILGXXNXGKSTLLNXLXXXXXXIXSXXXXTTXXXIXGXXXXXXX QXIFIDTPGLXXXKXXXXXLLXKXIXXALXXVDLILFVVXXXXXXXXDXXLXXXLX XXXXXXXLXXXXXXXXXXXXXXXXXXXXXXXXXXXXIVXIXXXXXXXXXXXXX XXXXXLXXXXXXXPXDXVXDXXXXFXIXEXIREKILXXXXXEIPYXVXVXIXXXXX XXXXXXXIXXXIXVXRXSQKXIIIGXXGXXIKXIGXXXRXXLXXXXXXXVXLXLXV K (SEQ ID NO: 6) and a stop codon
(g)MXXPKXXXXXKXXXXXXXGXXXXXXXVXFGXYXLXXXXXXXIXXXXI XXXXXALXRXVXXXXXLWXRIXXXXXXXXKPXXXRMGXGKGXXEXWXXXVXXG XVLFELXGVXXXXXXXALXXAXXKLPX (SEQ ID NO: 7) and a stop codon
(h)MXLLVAVSGGXDSXXLLXXLXXXXXXXXXXXXAAXVDHXXRXXSXXX XXXVXXXXXXXXXXXXXXXXXXXXXXXXXXXXXXXXARXXRYXXLXXXXXXX XXXXILTAHHXDDXIETILXXLXRGXXXXGLXGLXXXXXXXXXXXIXRPLLXXXKX EIXXXXXXXXLXXXXDXTNXXXXYXRNXIRXXLLP (SEQ ID NO: 8) and a stop codon
(i)MINXXIXXXEVXXIXXXGXXXXIXXXXEALXXAXXXXLDLVXISXXXXX PVXKILDYGKYXYXXXKXXKXXKKXQXXIXVKEVXLXXXIXXXDXXXKXXXXXX FLXXGXXVKXXVXXXGRXXXXXXLXXXVLXXVXXXXXXXXXXXXXXXXXXXXX XXXLLXPXXX (SEQ ID NO: 9) and a stop codon
(j)MXVXLXXLXXXXXXXGXXXXXXXPXXXXFIXXXRXXXXXIXLXXXXXX LXXXXXXVXXXXXXXXXILFVGTKXXXXXXVXXXAXXXXXXYVXXRWLGGXLX NXXTIXXXIXXLXXLXXXXXXXXXXXXXKKEXXXXXXXXXXLXXXLXGIXXLXXX PXXLXVXDXXXEXXAVXEAXXLXIPVVAXXDXNXXPXXVDXXIPXNXXXXXXXXL XXXXXXXXVXXXXXX (SEQ ID NO: 10) and a stop codon
(k)MXXLXLXXXDXXXXXXXNXXYRXXDXXTDVLSFXXXXXXXXXXXXX XXXXGDLXISXXXVXXXAXXXXXXXXXXXXXLXXHGXLHLXGYDHXXXXXXXX MXXXEXXILXXXX (SEQ ID NO: 11) and a stop codon
(l)MXXXXXXXXXXXRXWXXVDAXXXXLGRLAXXVAXXLXGKXKXXYXP XXDXGDXVIVINAXXVXLXGXKXXXKXYXXXSXXXGXXXXXXXXXLXXXXXXX XLXXAVXGXLPXXXLXXXXXXXLXVYXGXXXXXXAXXPXXXXX (SEQ ID NO: 12) and a stop codon
(m)MXXXKXXRXXXXRXXLLRXXXXXLLXXXXIXTTXXKXXXXXXXVEXL ITXAKXXXXXXXRXVXXXLXXXXXXXXLFXXIXXXYXXRXGGYTRILKXXXRXGD XAXXAXLELVD (SEQ **ID** NO: 13) and a stop codon
(n)MXXXXXXXXVKXLRXXTXAXXXDCKXALXXXXXDLXXAXXXLRXXG XXXAXKKXXXXAXEGXVXXXXXXXXXXLVXIXXXTDFVAXXXXFXXLXXXXXXX XXXXXXXXXXXXXXXXXXXXXXXXXLXXXXAXXXEXIXVRRIXXXXXXXXXXIX XYXHXXXRIGVLVXXXXXXXXXXXXXLAMHVAAXXPXXLXXXXVXXXXVXXXX XIXXXXXXXXXXPXXIXXXXVXGRLXKXXXXIXLXXQXFVXXXXXXVXXXLXXX XXXVXXFXXXXVGEGIXKXXXXFXXEVXXXXXX (SEQ **ID** NO: 14) and a stop codon
(o)MMKVILXEXVXXLGXXGDXXEVKXGYAXNFLIXKXXAXXXTXXXIXXX XXXXXXXXXXXXXXXXXXXXXXXXXXXXXLXIXXKXXDXGXLFGXIXXXXIXDX VXXXXXXLXKXXIXLXXXXXXXXGXXXVXLXLXXEVXAXLXVXVXXX (SEQ ID NO: 15) and a stop codon
(p)MXLXXLXXXXXXXXXXXXVGRGXGSGXGXTXGXGXKGXXARXXXXX XXXFEGGXXPLXXRLPXXGXXXXXXXXXXXVXVXXXXXXXXXXXXVXXXXLXX XXXIXXXXXXVKVLXXXXXXXXXXXXXXXXXXXXXXXXXXXXXXXX (SEQ ID NO: 16) and a stop codon

The artificial genes encoding sequences (a) to (p) are hereinafter referred to as "the artificial genes (a) to (p)".

The artificial genes (a) to (p) in the present embodiment may consist of any coding sequences, as long as the conserved amino acids are maintained, but it is preferable to consider the codon deviation, homopolymer length, and GC content in prokaryotes. When there is a plurality of codons corresponding to conserved amino acids, any of the codons may be selected, and an appropriate codon may be selected taking account of codon deviation, homopolymer length, and GC content in prokaryotes. Similarly, the stop codon may be an ochre codon (TAA), an amber codon (TAG) or an opal codon (TGA), but an ochre codon is preferred in view of codon deviation in prokaryotes.

In the nucleic acid molecule of the present embodiment, the artificial genes (a) to (p) may be arranged in any order. For example, in the 5' to 3' direction, they may be arranged in an alphabetical order such as artificial gene (a), artificial gene (b), and artificial gene (c), or in any order such as artificial gene (f), artificial gene (a), and artificial gene (k).

The artificial nucleic acid sequence in the nucleic acid molecule of the present embodiment comprises, as components (2), artificial intergenic sequences for linking artificial genes (a) to (p). The artificial intergenic sequences consist of non-naturally occurring random sequences of 10 to 60 nucleotides in length, preferably 30 to 50 nucleotides in length. Each of the artificial intergenic sequences comprises an independent random sequence for each intergenic region, and each may vary in length. The artificial intergenic sequences are random, but homopolymer length and GC content are preferably taken into account.

The artificial nucleic acid sequence in the nucleic acid molecule of the present comprises, as components (3), a front spacer sequence, and an end spacer sequence. Specifically, the front spacer sequence is added upstream of the artificial genes (a) to (p) linked by the artificial intergenic sequences, and the end spacer sequence is added downstream of the artificial genes (a) to (p) linked by the artificial intergenic sequences. The front spacer sequence and the end spacer sequence consist of non-naturally occurring random sequences of 200 to 400 nucleotides in length, preferably 250 to 300 nucleotides in length. The front spacer sequence and the end spacer sequence each independently consist of a random sequence and each may have a different length. The front spacer sequence and the end spacer sequence are random, but preferably take into consideration homopolymer length and GC content.

The GC content of the artificial nucleic acid sequences consisting of the above components (1) to (3) is preferably 30 to 60%. In addition, the GC content may be constant or may vary over the entire length of the artificial nucleic acid sequences. For example, the artificial nucleic acid sequence may have a GC content of about 30% over the entire length, or may have a region with a GC content of about 30% and a region with a GC content of about 60%.

Preferred specific examples of the above artificial nucleic acid sequences include the nucleic acid sequences of SEQ ID NOs: 17 to 22. The nucleic acid sequences of SEQ ID NOs: 17 to 22 comprises the artificial genes (a) to (p) in alphabetical order in the 5' to 3' direction, 42-nucleotide-long artificial intergenic sequences each consisting of an independent random sequence between each gene, and 271-nucleotide-long front spacer sequences and end spacer sequences each consisting of an independent random sequence.

The nucleic acid molecules of the present embodiment comprise the above artificial nucleic acid sequences and/or the complementary sequences thereof. That is, the nucleic acid molecules of the present embodiment may be either single-stranded or double-stranded. The nucleic acid molecules of the present embodiment are preferably composed of DNA, but each may comprise a modified nucleic acid of about 1 to 3 base pairs, e.g., at the terminus.

The nucleic acid molecules of the present embodiment may comprise the full length of the above artificial nucleic acid sequences and/or the complementary sequences thereof, or may comprise a partial fragment sequence thereof. The partial fragment sequences may be at least 300 nucleotides in length, preferably at least 1,000 nucleotides in length, and more preferably at least 3,000 nucleotides in length. In other words, the partial fragment sequences preferably comprise, e.g., at least 1, preferably 5 or more, and more preferably 8 or more artificial genes.

The nucleic acid molecule of the present embodiment may be a nucleic acid consisting solely of the artificial nucleic acid sequence and/or the complementary sequence thereof or a partial fragment sequence thereof, or may be a nucleic acid consisting of the artificial nucleic acid sequence and/or the complementary sequence thereof or a partial fragment sequence thereof cloned into a vector. The vectors which may be used in the present embodiment include, but are not limited to, plasmid vectors such as pUC19, pT7Blue and pGEM, fosmid vectors, BAC vectors, and the like.

The nucleic acid molecules of the present embodiment can be easily prepared by any conventionally known nucleic acid synthesis method.

The nucleic acid molecules of the present embodiment may be added to the sample to be analyzed at an appropriate timing. For example, the nucleic acid molecules of the present embodiment can be added to a sample before nucleic acid extraction, in which case it is possible to control the accuracy of the entire analysis process from genomic DNA extraction to assembly. Alternatively, the nucleic acid molecules of the present embodiment can be added to a nucleic acid solution extracted from a microbiota sample, in which case it is possible to evaluate the quality of the assembly alone. One specific type of the nucleic acid molecule of the present embodiment or a combination of multiple types with different sequences of the nucleic acid molecules can be added to the sample.

The sample to be analyzed may contain any cells, tissues, microbiota, or the like, but preferably microbiota. The microbiota refers to an aggregate of a plurality of microorganisms that exist in a certain specific environment, and may be composed of, e.g., at least 100, 300, 500, 700, 1,000, or more species of microorganisms. The species of microorganisms comprising the microbiota are not particularly limited and may be any class of microorganisms, such as bacteria, fungi, protists, viruses or the like, and may include not only known microorganisms but also unknown microorganisms.

The nucleic acid molecules of the present embodiment are standard nucleic acids that are compatible with general assembly performance evaluation tools based on single-copy marker gene information, such as CheckM, and are useful for accurate assembly performance evaluation in (meta) genome analysis.

According to the second embodiment, the present invention is a nucleic acid molecule comprising an artificial nucleic acid sequence of SEQ ID NO: 23 and/or a complementary sequence thereof, or a partial fragment sequence thereof.

The nucleic acid molecule of the present embodiment, as well as the nucleic acid molecule of the first embodiment, may be either single-stranded or double-stranded. Also, the nucleic acid molecule of the present embodiment, as well as the nucleic acid molecule of the first embodiment, is preferably composed of DNA, but it may instead comprise a modified nucleic acid of about 1 to 3 base pairs, e.g., at the terminus.

The nucleic acid molecule of the present embodiment, as well as the nucleic acid molecule of the first embodiment, may be a nucleic acid consisting solely of the artificial nucleic acid sequence and/or the complementary sequence thereof or a partial fragment sequence thereof, or it may be cloned into a vector. The partial fragment sequence may be at least 300 nucleotides in length, preferably at least 1,000 nucleotides in length, and more preferably at least 3,000 nucleotides in length.

The nucleic acid molecule of the present embodiment can be prepared and used in the same manner as the nucleic acid molecule of the first embodiment.

The nucleic acid molecule of the second embodiment is a standard nucleic acid having an artificial nucleic acid sequence in which the GC content is strictly controlled, and is useful for accurately evaluating assembly performance in (meta) genome analysis, in particular, for evaluating the effects of GC bias on assembly performance.

### [Examples]

Hereinafter, the present invention will be further described with reference to Examples. However, the present invention is not in any way limited thereto.

### 1. Design and synthesis of artificial nucleic acid sequences

### (1-1) Design of artificial nucleic acid sequences containing the artificial CDSs (SEQ ID NOs: 17 to 22)

Bioinformatics tools such as CheckM use a set of genes that are common in prokaryotes and exist in only one copy per genome (single-copy genes) as markers, and evaluate the quality of the assembly based on the presence or absence of the markers in the predicted genome sequence. Therefore, in this Example, an artificial coding sequence (CDS) that can be recognized by a general gene estimation algorithm such as Prodigal (Hyatt, et al., BMC Bioinformatics, 2010, 11:119) was generated from the 16 types of marker genes shown in Table 1 below.

**Table 1. Marker genes used to generate artificial CDS**

| [Table 1] | |
|---|---|
| TIGRFAM ID | Gene name |
| TIGR01079 | 50S ribosomal protein L24 |
| TIGR01024 | 50S ribosomal protein L19 |
| TIGR00082 | 30S ribosome-binding factor RbfA |
| TIGR02027 | DNA-directed RNA polymerase subunit alpha |
| TIGR02729 | Obg family GTPase CgtA |
| TIGR00436 | GTPase Era |
| TIGR01164 | 50S ribosomal protein L16 |
| TIGR02432 | tRNA lysidine(34) synthetase TilS |
| TIGR00168 | translation initiation factor IF-3 |
| TIGR01011 | 30S ribosomal protein S2 |
| TIGR00043 | rRNA maturation RNase YbeY |
| TIGR01066 | 50S ribosomal protein L13 |
| TIGR00059 | 50S ribosomal protein L17 |
| TIGR00116 | translation elongation factor Ts |
| TIGR00158 | 50S ribosomal protein L9 |
| TIGR01071 | 50S ribosomal protein L15 |

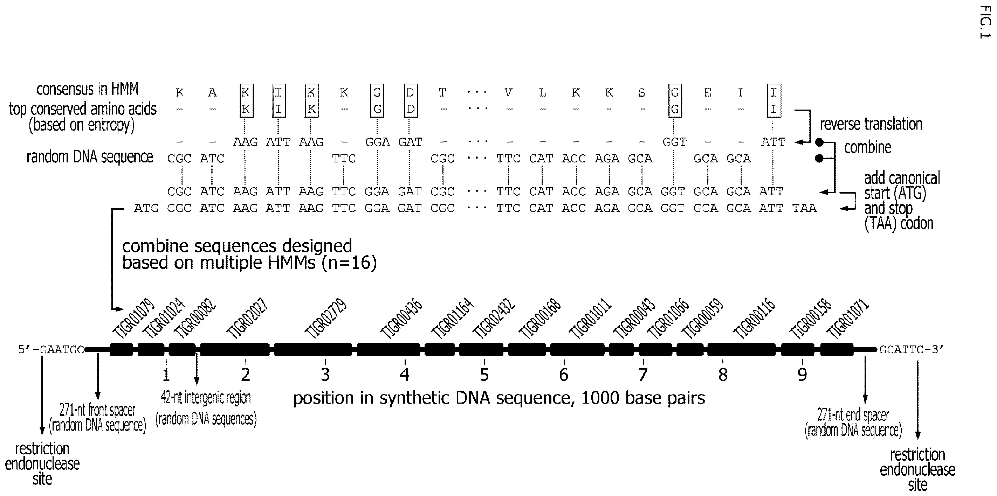

Conserved amino acid residues in the consensus sequence extracted from each marker gene based on a hidden Markov model (HMM) were searched for and reverse translated into the corresponding DNA sequence (3 nucleotide codon). The remaining parts of each marker gene were replaced with DNA sequences encoding random amino acid residues, combined with DNA sequences encoding conserved amino acid residues, and a start codon (ATG) and a stop codon (TAA) were added to obtain artificial CDSs. An artificial nucleic acid sequence of 10 k nucleotides in length was generated by linking the artificial CDSs with random DNA sequences (intergenic region). An outline of the procedure for generating an artificial nucleic acid sequence is shown in FIG. 1.

Six artificial nucleic acid sequences, seqHMM3501, seqHMM5001, seqHMM5002, seqHMM5003, seqHMM6001 and seqHMM04, were generated, which have the same arrangement of the artificial CDSs and the conserved amino acid residues encoded by the artificial CDSs (see SEQ ID NOs: 1 to 16) but differ in other parts (random sequences).

seqHMM3501 (SEQ ID NO: 17) [Formula 1A] 
GAATGCACAGAATATCTTACATCGGTACAAGAATTCACAGAATCGAATCTCAACAGAATC
AAAGCACGAATTCATCAAGAATCACTTATCAAGCCTTATCTACAGAGAACTTTATCATCA
GATTCCATACAGAGTTACAATCAGGTATTCACGTATTCAGACTTACTATACGCATTTACA
GATCGTTTCAAAGAACAAGAAGCTTCTATACAGTTTCTCACGAAGTTACTTTCGACAACT
TACAAGAAAGTCATTCTCACGATTCAAGATATACAGAATGTACTACAAGATTAAGGGAGG
AGATTCAGTTATAGTTATATACGGTAAGAGTAAGGGTGAAATTGGTATAGTAGGAATAGT
AGTCTACTACTGTATCAAGGTGATTGTAGAAGGAGTTTCAAGTTCAAAGAAGTACGAAAA
GATCAACATTCATTCGTACGAATCCGGATCGTATTCGTCATCAGAACGACCAATAAATAC
AAGTAATGTTAGAATACGATCAAACATATTACACGATACTTACAGAGTTGACTATAGAAA
CTTATATCACATCAGAAAGAGAAGAGTTCAGTCTTACAAGGGAATATCGATTTAATCTGT
CATATTCACAGCTCTATTCCTCAAGAACTTCACGTCGATGGTTTCGAACATAATTTCATT
GTTCCGAATATCTCGAAACGACAATCTCAACAGTATATTCTATTCGGGTTACACGGTCAC
AGTTGAATTCATCATTAATGAGGGTGAAACTGCCAGAATCCAGGGATTCAATGGAATAGT
TATTAGTATTCGACTCTGTGGAAACTTCTCTGCTATTTCGGTGCAAAAGCAGGTACAAGG
AGACGGTGTAGAGAGAGTTTTCAATCTATACTGCTATTACATAGCTTATATTCTGGTGTC
AGACCAAGGCCTTGTTGAAAGAGCTATACTTTCCTACCTTAGGTCAGTTACAGGTAAGCA
ATTAAAGATAAAGAGGCTCTATTAAGACATCAGTTACTGTTATATATACGAAGGATTCCG
TACCTTAATGATGGCATACTACAAGAGATACCAGAGAGTAAGAGTCGACATAGAATTCAG
TATTAAGAAGCTGTTATACACGTATATTGTTGATATCGTATATATCCTGTACCAAGTGTG
GACAGTTGAAAGTTCACGAGATTTGACTCTGGAATTCGTATTCGTAACGTTATTATACGA
CGTTATACTGGAAATATCAGTTGTATTCGGACTGCACCTGGCTATAGGTTTCATAATCAG
AGAACTAAGTACGAAGGTCCGATTGTACTCAAAGCCAAAGCTTCTATTCGAAAGTGACGT
CTCACTGCATACAGATCTGAGAATTCAAGTCTTGATAAATCAGTTAATCTGTATTTAATC
GTACAAGAGATCAAATATTACGAGATTCACTGGTTACGTCATGATTTACACATTCTATCA
AAGACCTCTTGTACATGGAAGAGGTTATACGCTTGGCTTCAGTTTAAGGAGGGTACTTCT
TGACTATATTAATGGATCTGCCATAAAGTACCATATTATAATACTTACTTACAGCGAATT
CGGCAACACAGATGGTGTATCTGAAGATGTAGACAAGATAATAATCAATCTGAAGTACTT
ATTCTATTACTGTCTCAGTTTCTCGACGAGTATTATATACGGAATTATCAAGTCTGGTAA
TTCTCAGCATTACGCAATACAGGTATACGAATTCTCACAGTCGGTAGAGGTACAAGCATA
CGACAAGTCAATTAGAGACTTATATTCGATATACTATTTGTCTATCTACTTAACGGTTAT
TCGAGGCTACGGTTATTTCCAGTCGAATTTACAGGACTCTGCTTCATCGATATTATATCT
TATTAAGGTTGATGCAGAATTCGATCCTGTTTACATAGTTTCCTATAGGGTAATCTGCAA
TCCACATCAGTATTTATATTACAAGGATTCTCTTATCCTTTTCATTAGCACTTATATATC
AGAAATCGGTCAGTTCGCATTGGAACGCGCATTATTATATCTTGATCAAAGCCTTACTTC
CATCGATCGATTATTATTCAGACTTTCTCAGAGATTCAGATATCCGAAGTATTACAGAAT
TCACTACGCATTATCTTACTTCTCGAAGCGTTACATTTCAAAGCTTGATTTGTCATACAG
ATACTGCAATTGTCTTTATCACGTACAAATTGATTCTCTTGAAGAACTTGTTCTATTCCA
GGATATTCGTCTTTTCCAAGGATCAAATTTGGGTTTCAAGTCAGAGACGGAAATTTTCCG
AAAGTTGAGACGATATTCGCTTATCTTGCTACGTTTATAACTTCGATTCAGAACTACATA
CGCAAGAATTCGTTTATCTGCTATGTTCCAAGATGACGATTTCGATCTAGTATTATCGGG
CTTCGGTGGATCAGGTCTCGAAATATCCGTTATCGAAGAATACTTCAAGGAAGGAGGTCC
AAGAGGTGGAGTTGGAGGCATAGGTGGTTCGGTATACAAGGATCAAGTACTATCTATTAC
AGACCTACTCGTTTATTACTGCAAGAGAATACAGAAGGCAGTATACGGTTCGAATGGATC
ACAGGTTATTACGGAAGGTTTCTATTACCGACAATTATCAATAGACGTTCCATCGGGACA
TATTGTGATCACGTTCCATATCAAGGTATCTCTGTTACAAGTCAACAACGAATCAAAGGA
 [Formula 1B] 
TTTACAAGAGAAGCATGGTGGCCACACAGGACATGGCAATGATATCTTCAATACAGCTTT
ACTGTCAGACCCTTTCCAGTTATCCAGAGGTATTCACTATCATCGTTCTACGTTGATCTT
GCACTTGCGTTATTTGGCAGATGTTGGACTTGTTGGTAGTTCAAGCTGTGGAAAGTCACT
ATTATTGGATTACAATTACGATCAAGGATTACCAATAACGATTTATCCATTCAAGACCTT
CATACCAATTTTGGGAGTTACAAGACGCAAGGTATTACGATCATTCTGCAACGCTGATAT
CCCAGGCTTAATTGTACTTTACATCGCAGGATATGGTTTGGGAAATCACTTCTTAATTCA
CATCAACGCACTTATACGATTGCTGTTCTTGATATTCTTATACGCTGTTCTTACTTACAC
GTATACTCAGTACATTTATCAGCATCGGTATATTCTGGAAACATATTACGAAAGTCTCAA
GCTACCTTTATTCCAGAGGATTCATTCCAAGGATGATTACAGCTTCAATACAAGAAAGGT
TATACTGCAGATTCTATCAATCGGCAGATTATACATCGTCATTACTTTCAAGAAGCTACT
CCTGTCTTTACAGAAGCATCAGATCAGACAAGAATTCTCAGATACAAACTGTTAATGTTT
ACTTCAGGATTCAGTACCAGTATATCAATTCGCAATAATGACGAGTGTTGCAATATTGGG
CTCTATAAATCAAGGTAAGAGTACGTTACTTAATCTCCTTGCATTATTATCGCATTTCAT
TTTAAGTACAAGGATTACCACAACATATTTCACAATTTCGGGTATATTCGAGTTCCTTCG
ATTCCAAACTATCTTCATTGATACTCCAGGATTGTATCGTTTCAAGTTCCAAGCATCGAT
TTTACTTCCAAAGCATATTCAAAGAGCTCTTTCGTTCGTCGATTTGATCTTATTCGTAGT
AATACTTACTGTATACAAGTCACGTGATTCAGTATTATACCTATCGTTAATTACTGTATC
GTCTTACATACCACTCTATCGAGAACATACGATCTTATTCCAGATCAGGAGCTTCACAAA
CTTCTGGAACTCAATATTCAGTCAAATCGGTCAATTACACATTGTTTTAATAAAGATACA
GAAGGACTACCGTATACTCAAGGACATTTATCTCGACAGAACAATCTTACTGTATATTCG
TATACCAATTCCAGTAGATACGGTTCAGGATTTACACAAGATTTTCAAGATTTACGAATA
CATAAGGGAAAAGATTTTGATTATCACAGATCGTGAAATACCGTATTCAGTTCCAGTATA
CATACGATTACCAAGTTTATTCCGGACACGATCTATTTATATAGATATCCGAATATCTGT
TATCAGAAGCTCCCAAAAGTCTATTATAATCGGATATCTAGGACGAAAGATCAAGTACAT
CGGAAAGCTCGTTAGATCGATTTTAAAGTACATATCAAACGAAGCTGTATATCTTTGTTT
AGTTGTTAAGTAACTATTCTGGATCCATTTATCGATCGAAGTATCCATTTCAGTTATGTC
TCACCCAAAGCAGATACAGTCTGTAAAGAATCGAAATATCTGTCTCACTGGCAGTTCACG
CAATATTACAACAGTAAGATTCGGTTCGTATCCTTTACCAGACTTATTCACTGTACAGAT
TTCGCTATTACAGATCGCTATCCAAAGATTCGCACTGATCAGGTCGGTCATCTTCTGTGG
CACCTTGTGGAAGCGGATAGAAGTCGAAAGTCTATATACAAAGAAGCCGTCAGATTTCAG
AATGGGTTCCGGAAAGGGCCTACGAGAAATCTGGTACAGAGATGTCCGGATTGGCTTCGT
TCTTTTCGAATTATCTGGTGTATATCACGGTCATTTATACTACGCACTTTCTGCAGCGAA
GAATAAGCTCCCATCTTAAATTTCGAAGTCTCGAGTCAAGAAGCATCTGCCAATTTATTC
AATGTATCTTTTGGTAGCGGTTAGCGGTGGTATAGATTCACACTACTTGCTGAAGTCGCT
TAATATATCCACAAAGTTCTCGCATTTACAGAAGTCGGCAGCTCGTGTAGACCATACTTC
GCGCATTACCAGCTTATCCAGTACACTTATAGTAAAGGCTTTCCTATATTCGGATACAGA
TACACAAGAACAAATCTCTGGATTCATTCGAGAACTTCAAGCTTCTATACACAGAATACG
GGAAATTTCGGCCAGAACGAATAGATATTACTCTTTGATTCGCATTACCAGATTATACTG
GATATACTGTATACTTACTGCTCACCACCGAGATGATTCAATAGAAACAATATTAGTTTC
CCTTTATCGGGGTTACAGCTCTACAGGCCTTCAAGGATTAGCACATCAAAGTTTACGTAC
ATCTATCCATCGTATAATCAGACCACTTTTGTTCGTTCGTAAGCTAGAAATAATATCAGT
TCTTCAGAATATACGTCTTCAGTTCCAAGGAGATATAACGAATCTGGAAACAAAGTATTC
AAGAAATCAGATAAGGTATCCACTTTTGCCATAAGACAAGGTTCTCAATCTGTACAAGAA
GTTACAACCGTACTTCATGATAAATAGAGGAATTATTATACGAGAAGTTCAAAGAATTTA
CGCCATAGGCTTACATTATTACATCAAGTCATTCTCCGAGGCTTTGCGAATCGCTAAGTT
 [Formula 1C] 
CCAGATCCTTGACTTAGTCTTAATTTCGTCAGATCAGACATATCCAGTTCGCAAGATATT
AGATTATGGAAAGTATCTATACAGAATCTTCAAGATACCTAAGAATCGTAAGAAGTACCA
GCTGTTCATATCCGTAAAGGAAGTCAAGTTGCGATATATAATAGAATCTACAGATTTCAT
CCGCAAGATATCCGAAAGTATTACATTCCTACACTTCGGCTCGCAAGTTAAGAGTATAGT
CTCAGATCGTGGAAGACTATTCGTACATCAAGCATTACACTATACGGTTTTGTCTACAGT
CACAAGAAAGCTATCTTTACCAACAGGAGTTATCATATCGTTCCTCAGTATCATTCTCAA
GGGTCCAGTATTACTTCTTAACCCTGTTTACGCATAAAAGTCTTCGACTTATCGATTACA
AGAAAGTCTACGAAACGAAATGAGTGTACAATTGCTATTATTAAGGAGTACTCTTCGAAA
GTTCGGACGGAAGAATTTACTACAGACACCTAATCAGAGTTACTTCATACACAGTATCCG
CCATCGGATCTATATCATCTACTTATTACTGGGAAACTCAACATTACATCAGATTATTAC
TGCGGTCTTCTTCTGTTACGGAATACTGTTATCAATACTTTTCGTTGGTACGAAGTCAGC
TTCATACTGTTCTGTGTCTTACGCAGCGCAAAGAGTCTGCTCACTTTATGTCATATCGAG
ATGGCTCGGCGGATCTTTGACAAATATCATCACAATAAAGGACTTCATATCGAACTTGCA
AACTTTGGAGAAGTTCATCTGTTCCTATCATATCGAGCAGAACATAAAGAAGGAATCAAT
ATACGTTCGATCTCAAGGTCCATACTTAACAAGATACTTAATAGGAATACAAGATCTTCA
GTTACAGCCGCTTATATTAAACGTGATCGACAAGTTACCAGAACAGGATGCAGTAAGTGA
AGCTTCACTGTTATTCATTCCTGTAGTGGCTCTATTCGATTCCAACGAGCAACCTATCAA
CGTAGATATACGTATACCGTCTAATACATCGATATACTCAACCGAAATCTTAATTTCAAA
GGCATCTTATCTGTTAGTCAAGGTTCAACGTATTATTTAATGGAATCAAATTCTGTCAGC
TTATTCAGCAATATTCTCGAGAATGATTCGATTGTACCTCATTCAAACAGATAACTTCAA
GCTACAGTTCCAAAATGCTCAATACAGGCAAGGAGATCAAAGAACAGATGTACTTTCTTT
CATATACGAGTTCCATCTATTCCAGGCTTCGATTATACAGCAGAATTACTGTGGAGATCT
TACAATAAGCTCAATTCAGGTCTACAATTTCGCATTATCTTACAAGAAGGTATTCAAGGT
CCTTTCCAATATTTTATATCATCACGGTACATTACACTTGACAGGTTATGATCATTCTTT
CAGTTCTACAAACGATTTCATGTTCGCTGTTGAAATTACTATACTAAGATATCAACAATA
ACATTCTACCAGAAGTACTCTTCGTACTATACTGTACATATTCATGATACTGGAATATCA
GCTGTCGAAGTATCAATTAAGAACTTGGCGCAGTGTGGATGCTCAAGGATATTTATTAGG
TAGATTAGCTCGCTTCGTTGCTTCCACATTGATAGGAAAGATTAAGTTATATTACTCACC
ACAGAATGATAAGGGAGATCAGGTAATAGTCATAAATGCTACTTCTGTGTTCTTATTAGG
AGTTAAGACGAAGGCAAAGATATACATATCTGGATCTTACTGTCATGGATCAGACTTCCG
TTCATTACTTCGATTCTTGCTCGAAATTCCAGTTCTACCAAAGCTTATTCGAGCGGTTAA
TGGAGTGCTTCCTAATACGTCTTTGTATATCGAATTCACATACCGTTTGATCGTTTACAC
AGGTTTATCATTCGAATCTCTAGCTATTCTTCCAGACGAAGTATTATCATAACGTTATCT
GTTACGTATTTCTTCGGATCAAATCAAGTCAAAGATGAGATTCACAAAGATCGTCAGAAT
ACAATCAGTCAGGTTCAGATTACTTAGACTTCTGCACAAGGTTCTACTTGCTCATATTCG
GATTCTAACGACAAACAACAAGTACGACATCGATCTATCTGTAGTAGAGTATCTTATAAC
ATATGCAAAGATACCAGACTTCTTCGTTACAAGGTATGTACAAAGTTTCTTATCTGGAAC
TCTTTCGTATCTGTTACTTTTCCAAGTCATATCTGCAGATTACCACGTCAGATTCGGTGG
ATATACAAGGATATTAAAGTCGTTACGGAGGTATGGTGACTCAGCACAAACAGCGATCCT
TGAGCTTGTCGATTAAGTTCTACAAATTTCGGCATATCCTCGAACAAGTCTTTATATTAT
GACGAATTCGGCTATACAGAACTCTGTAAAGATTTTGAGAACTGTTACGTACGCGAAGGT
ACACGATTGTAAGAGTGCCTTAAAGGTATCACAAGTTGATCTTGATCATGCAAGTATCTT
ACTTAGGTTCGAAGGAGATCATCAAGCGACGAAGAAGTCATATACGGAAGCACTAGAAGG
CTCAGTATACTGTTTCCAATCGAACAAGACAAGTTATTTGGTTTGTATTTTCTGGATAAC
TGACTTCGTTGCCTCAAAGAAGGGTTTCACAACGTTAAAGCAAGATTTCTGGACATCAGA
 [Formula 1D] 
ATTCCTTCATACAGGTATTACGATTCCTATACGGAATCATTCGATATTATCGGTCATCAT
TCAGATTATCTGCCTCGAATCTCTCTTAGCTCTTCGCAAGGAGTTCATCCAAGTTAGAAG
AATTTATTCAAGACAGTTCGCTTTATTCATCAAGATTGTCTTCTACTACCATCGTATTCG
CAGAATTGGCGTATTGGTCATTCACCATTACGGATTCAAGTATCATTTCACAAGGACTTT
AGCGATGCACGTCGCTGCGCTATACCCTCAACAGTTGACCGATATTCGAGTATCATTCCA
CAAGGTATATACACTTTCGAGTATATCATTCTGTCTACTATCGAGCGTTATCAATCCGTA
CTCAATTTTCACCGATATTGTTTGCGGAAGATTGTATAAGTTCCAGATTCTTATCTTACT
GAGGCTACAGATATTCGTCGTTATACAACGCACATTCGTTATACCGAAGCTTTTACGTAT
CAACACTTCAGTGATTCTTTTCCAGTATACGACGGTAGGAGAGGGAATAGTTAAGAATCA
CAAGAATTTCTCGCAAGAAGTAAGCTTATCGTACAAGGAATAATCATCAACGTCTTTCTC
AAGTCAACAGGAATTACAGTTATCTATGATGAAGGTGATCCTTGCAGAAGTAGTGTCGAG
ATTGGGAAGTATAGGCGATACAGTCGAAGTCAAGTATGGTTATGCCATTAACTTCCTCAT
AAATAAGGCTACTGCGCCGTTATATACATATCGCATTATTGAAATCAGAATCCTTACAGA
GATTATTACGAACACATTACGACGAAGTATATCTGTACAACTCTGTTACGAATACTTCCT
TCTGCAAATTCAGCTTCTTTATATTTCGTACAAGCAAGATGATCAGGGATACCTGTTCGG
TTCTATTAGGATCATTATTATACCAGACAAGGTTTATTCAAGCGAATCAGTTCTGCGTAA
GATATCTATAATATTGAGGATCTTCATATCGATCTACAAGGGAATTACGTTCGTGAGTTT
AACATTGATTTACGAAGTGAGCGCTATTCTTGAGGTTCATGTACGAGTACTTTAAATCGT
CATATTACATCACGATTACATACAGTCATCGTCTTACATGTATTTGCTTATCTTGCAGTT
ACTCTGTCAATCGTTACGTTTATTCACGGAAGTAGGTAGAGGCCTGGGCTCAGGTATCGG
AACAACAAGCGGTGTTGGTTACAAGGGCTACAAGGCGAGATCTCATCAACGTTATATCTG
TATCTTCGAGGGAGGAAATCGACCACTTATCAAGAGATTACCTCTGTCTGGATCGTATAT
CCGAGAAGGTTTATTCCAAGTTCTTGTAGGAGTAATTACTCGAGGTTTATATCGTATTTC
GTTACTTTCGGTTTCATTACAGACCCTTCATTTACTATTCTCGATTACAACATCGTTCAG
TACAGTAAAGGTGCTATTATTCGAACATATCGTCCAAGAAACTCGCTATTACGTTATATT
CTGCTTACATCAGCTTTCAGTATCACATATTCCGATTATTTCTCTGCTTATCTAAACTGT
ATATTCCATACGACAAGATTCAGTTTCAAATTACGAAGGTCTACTATTTCACGAGTCAGA
ATTCTATATCGAAAGCATTTCTCAGTATTCCAGTCTTACAGCTTTATTTCATCTGTCTTC
TGTTCAAGAAGTTACATCGCAACTTTATACTGCAAGGAACAATCTATACACATCGTTCTT
ATCGAAATTACACGCTCAAGTATATCACAAACCGATTTCTGTTACTATTCCTACAGCGTA
TATTACAAACGACATTCCAGAAGATTGCATTC
seqHMM5001 (SEQ ID NO: 18) [Formula 2A] 
GAATGCCACCGAAGTACAGGCTCAATCGCGACCAATTCATACCGATCTTCGTACTCCACT
CGACATCCGCTACCTATATCGGAGATTCAACTCCGGCTTCATACTGTCCGCAAGTTTACC
CGCATTTACCCAAGCGAGACGTTATCCGACTCGATCATCTCACAGTCACTCCACCGAGAC
AGAATACACGAGGACTACGAATCTGTCAGCTTCCCTCTGTCACTTACATCCCGGAACTTA
TTCTCGGGACTCGGAACTTTCAGATCCGGCCAAGATTATGCGCATCAAGATTAAGTTCGG
AGATCGCGTCGGCGTGATCGTCGGCAAGTCTAAGGGTATCAGTGGTGAGGTCGTCAGTGT
CCACTCAGTCAGAGAAGACGTCATTGTGGAAGGCGTCCGGCACGAAAAGAAGCTTCAAAA
GGCTACAGGCCTTTCTGTCCCGAAGGGAGATACGAAGAATCCAGAACAACCTATCTCGGT
ATCAAACGTGATATTCATCGGCATCTCACAGCAACTTCCAACGCACGTTAAGTCACGCTC
CTCAACGAACCCTTACAAGGGACGTTTCCATACCAGAGCAGGTGCAGCAATTTAATTCTC
TGTCGACATACAGGGTCGTCATCCAGGACCATTCCTCATGGTCTCGAAGATTGCACTGCT
CCCTGTCAGCGAAAGTTATTCACGCCTACCTCACGTATTCTTCGGTGGTCTACGTGTGTT
CGTTCGCGAAGTAATCCACGAAGGTAGAGTACCTCGCAATCAGTACTTCCGCGGTCTGGT
TATTAAGAGCGTACAAATAGGACAACCAAGCCATACGGAAGTTCGAAAGACACTTCACGG
CCGAGGCGTGGAACGCGGTTTCACCACGAAGGGATCGTTCATTAAGAGTATTTCCGTCTG
GTATCGGGGCAGTGTTACGCGAGCAACGCTAGGTTACTTGCGTACACGCACAGGTAAGCA
TCCAAAGATCAAGAGTCGAGAGTAACTACGAGACAGCTGGAACACTTACACGCAAGCAGT
ACATCGAATGATGGCGTGCATCTACCGCCTGCGACGCGTATATTCGCTTATTGCTGGATC
TATTGTACGTCCTCTAACCGTCTACATCATCGATTTCCGATCTACGACATACGAGGTTCA
ATTCGTAGAATCTTCCGCCGATCTTACCTGTGCGCCGGTCTTCGTGGTCTACCTGTTCGA
CACACTACATCAGGACAGACGCGTTTTCGGTTTAAAGTTCGCAACAGGATTCATCCGGAG
AATCCTCCGTATACCAAACAGCCTCTTCGGACCACCATACCTTGACTTCATACGAGATCG
GTCTCTGCACGGAGTTCCACGTATTAACTCACTGATCTGCCTCCTGAGATACTCATAAAT
TCCACATCATCAAGGCTTCGGCACACTTCATTTCCGGTACATGGAAACCTCATTCTCTCC
TCAGCCGCTCAATCTGGGTTCTGGAGTTACCCTTGGTCTCGTCTTGCGCCGCGTTCTTTT
GAGAGGAATTTTAGGTGACGCGATTATTCGTCTCAATATTTCCGAGGAGTTCTGTGAGTT
CACAGTCTTCGGAGGTGTTTCAGAAGATGTACACAGAATCATCGGCAACCTCAAGTTCCT
GACCGCTTCCAGGTCACACGTCCATCGGAAGAACTATATCGACTCGAGTCTCCTACCTGG
ACAGAAGCATCTCGCCATACTCCAAGGTCCGTCAGTCCGACAAGTTGAAGTCCGAAATCT
GTCAGCTGGTATTAACCCGCTCGATTTCGACAACTCTCTGCTCATCACCTTATCCGTGCC
GAGTGGCTCAGGCTACAGTCTCGAGTTCCAACTGCGGCGTCATCTACACAAGCGATACTC
AATTAGGGTGGATGCGGAGTTCTCACCTGTTCGGAGCGTTTCCTACACTGTGTTCAAGTC
AGGTACAGTACCTAAGCGCAATTCGGATTCGTTGAGGCTTCCAATTGAAACGTATCACGG
ATCAACCATCACCGATGCTTTACATCTCGCAAGAACACTGCTGCGAAGACGTCTTAAGCG
TTCGTACCACGACAATCCTATATCGTACCCTGCTGTCACATCTCGGAGCACACTTTCGCT
TATCGTTCGTCCAACATACCTCACAGCGGTCACAATTCGTCTACTGGACCTCTCCTTCCG
TCTGGCTAATTGCCTGGACTGCTATTCCATCAATACATTGTGTGAATTAGTCTCAGTCCA
GAGCATCAACCTGTTCCAGACTACTAATCTGGGTGTTAAGAGCCAATACGAAATCGACTC
GAATTTATCCAGAGCTATTCTGCGCTTAACGGATACCTAAGTTCAGTATCGACGAGAAGT
ACGGCGGATATCTCTGCAGAAGATGTTCGCGGACGACATTACGACCGACGTCGTCTCAGG
TATTGGTGGCTGTGGCTTCATCCCTGCCACACGAGAAAACTATTACGAGGACGGAGGCCC
AGAAGGTGGTTCTGGTGGCCGGGGAGGAGAAGTAACTCCACAGATCCACACGGTTATATC
CGCACTCGTCTATCCAAACTCGTTCCGGGTCCTTCAAGCGGCTCAGGGCCTACCGGGAAG
GCTTTCGGTTCTCATCGGCGAGAGGCATACCAATCGTACTATCTCGGTCCCTGTTGGCAC
GACCGTGACTTCGTACATCAGGCAGACGAGACAGATTCACACACAGGCGTTCACTACACT
 [Formula 2B] 
CGGAAAGCGTACTACAGGTGGTCAGCGAGGTACCGGCAATCATTTCTTCCGATTCACGGT
ATTCTGGGTACCGCGAGACGGATTCACGGGTTATACTCCGCAATCGACATCTCTCGGCCT
TTATCTTACAATTCTTGCGGATGTGGGTCTTGTGGGCGCCATTTCCAGTGGCAAGTCGGT
CCTGTTAAGGCGATTATCGCCAGAGGATTTCGGTATCCGAGCCTACTACTTCATTACAGC
ATCCCCGACCCTGGGTCGTCCGACATTCACCGAAATTCGCAACTCCTTCGTTGCCGATAT
TCCGGGTCTGATTATCTCACGAAGCTGCGGTTACGGACTGGGCCTACATTTCCTTGGTCA
TATTACAGGCAGACTTTCGCTTTCAACACTGATTCTCACGTTCACCAGTCCGAGTACACG
TTCAACATTCCCGGGAGGATACTGTCAGTTCGCACTGGCAGATTACAACTACGGACTCAA
CTGGGATTACGTCTCCACCAGTTTCGAAAAGGCAGATATCGTCACATCGACGGTACACTC
TTCGCCATTCACGGGAACTGTACTTCACCACGTCGTTCTCTCAAGGACGTACAGCTGGTT
CACCTACCTGGGACTATCGGATTTCATCGCACTCGTACGCATCCAATCGTACTAAATCAG
AGAGCAACTCTCGAGACCGATCTCATACTTCCTCTGCATGCCTCGTGTCGCGATTCTAGG
TACAGATAATCTCGGCAAGTCGACTCTGCTGAACGAGCTGGATTTCCAACATCCTGGAAT
TTGTTCCGCTTCAGGATCAACGACGACGCTACTAATTATCGGAGTATCTCAGCCTTACAA
GAGCCAGCTAATTTTCATTGACACCCCGGGACTGCTCTCAAACAAGCACTCAACGCTCGA
GCTGCTGCCTAAGTCAATTCCAGATGCTTTGCAGGAGGTAGATCTGATACTGTTCGTTGT
ACCAGTCTCTTTCGACTGGCACCTGGATTACGTTCTTACAGCGTTACTAAGACTCGACCA
GGGATACTCTCGGCTGCACACGCTTACAATACCGGAGACCAGGAATCTCGGTTACTGTAC
ACGCTCTTACAGTCAGGCTACCAAGGCAACCGACTATCGAATTGTGGACATTCAGTCACG
CAATTCTCTCTGGACATACCACACTGCGATCACGTACTGTCCGAAGTTAATCGGTTCAAT
CCGACAACAGCCTAGTGACTCCGTAACGGATCCACTGTCAGAGTTCCGAATCCCTGAAGC
CATTCGTGAAAAGATTCTGGCACTACACTTCTGCGAAATTCCGTACAGTGTGCATGTGCA
GATCGAAATCGGTTCAACGTCTGCCGTCAAGGTATTCACTATCTGCAAGGCGATCATCGT
ACCTCGCCACAGTCAAAAGTTCATCATCATCGGCTCTGCTGGAATCAGAATTAAGGTTAT
CGGTCGCAATCAACGTCCGTTCCTGAAGAGCTCACTTATTTCCCGCGTGTTCCTTGGATT
ATACGTCAAGTAAATCAAGATCCGTCTGTTACACGCCCGATTACTCAAGGCAGAGATGTG
TACTCCGAAGGTCGGAGACTGGTATAAGGAAACGGATTTCCGGTTACATGGCGCGAGAGC
GACATACGCAAATGTGATTTTCGGTGTATACCGATTGGCGATATACACCGTACTCAACAT
AGGAAATTCCCGCATTTCCGAAGAACGTACAGCTTTGCTCCGTACCGTTGTACCTTGCGC
CATTCTGTGGCCGCGTATCATACCTATCACGAGGAGCCAACGAAAGCCGGCAAGTCAGCG
CATGGGTCTTGGTAAGGGCTGTATTGAATCTTGGATCAAGTCAGTGGAGCTGGGTAGCGT
TCTGTTCGAGTTGGATGGTGTGCTACTGCAGGGATACGGAACAGCTCTTACAGCCGCTAA
GCAAAAGCTGCCAGTTTAAACGAAGTCTATACCACGAGCTTCTCTCTTCGCTCAGAGCTA
CATGCGTCTTCTTGTCGCTGTAAGCGGTGGTCTGGACAGCGGAGGTCTGCTCACTCCTCT
GACGAGGGAAGCACTTCCATTCGTACGAAGGTCGTATGCAGCGCGCGTTGACCACCACCA
CCGTGTTACCAGCGGACAATCGCAAGCCTTCGTGGATCTCACGAGATTACAGGCTGCGAT
CGCAAGAAGGAAGGTCGTTCGAAGATACCAGGCTCTTCCACGACCTTCTTCAACGACTGT
AGAAATCGAGGCACGTGTCGTCCGCTACTCAGCACTGTACATACAGGGACACCCTTCCTC
GCACAAGTACATTCTGACGGCGCATCATCAAGACGACCGCATTGAAACAATCTTAATTCG
ACTGCATCGCGGTCGTCGAGGACGTGGACTGGGAGGTTTATCGAACGAAGCATCCGGCTC
AACCCAAGTCCTCATCTCTCGTCCGCTCCTAGGTACCCGAAAGACTGAAATACAAATCTT
CACCGACAACACGCTCCTGAGTACCTACCAGGACTTCACAAATACGATCAGGCACTACTA
CCGTAACCGAATCCGCGATCTCCTGCTCCCTTAATCAGTACAGCACACGGTTACGAACGT
TCATACCGAGGCGAATATGATTAACTCTTCAATCAAGGATCCTGAAGTAGGACGAATAGA
GGGATTCGGTCTTCGTCTTCAAATCCTTCGGAAGACGGAAGCGCTGTACTTCGCACGAAC
 [Formula 2C] 
CATTCCACTTGACTTAGTGCGGATCTCGTATCAAGGTAACGTTCCTGTGAGTAAGATACT
GGATTACGGCAAGTATCTATATACTGGAAACAAGTCGCGAAAGACGTCGAAGAAGTTCCA
GCAACTAATCCTGGTTAAGGAAGTGCAACTGATACTTCGTATCCTGTCAGCTGACCGCAC
AAGCAAGCTCGTTCTCCAAGACGTATTCCTCGTCGTTGGTCAACTTGTGAAGAGTCGAGT
ACGCTTATCAGGTCGAACCGCATCGATTACGAACCTTAACCCAGTCGTTCTGGTCATCGT
GATCCACACAGTTCGTTCGAGCTCTCTATCTGTCAGGCATACCTCCATCTGTTCAGAGTA
CCCTACTACCCTGCTTTTGGCCCCGTCTGCACATTAATTCCTCGAACTTCGGTCAGACCG
ACCATCAACCGATTTCGAAATGATTGTGTACCTAGTCACGCTGTTACGCGTATATCGAGA
TCACGGTCAGTACGAGAGCTCGGGTTCCCCGTCGTTATCCAGTTTCATTTCATTCGCACG
CCCGTTACGGATCTTCATTAGCCTGCATCACTGGGAGTCGGGATTGCACAGGATATCTCA
TCAGGTCTCAACTGCCCGCTGTTACTGCAACCACATTCTCTTCGTTGGCACAAAGGCGAG
GAACCTCTACTTAGTGTACTACTCCGCAATTCTCGTACCGCCTACGTACGTCTCGTTCCG
TTGGCTCGGCGGATACTTACCGAATCGGATAACGATTGCTCAAAGTATTGGATCACTGGC
ACGCCTGCACGAAATACACGCTTTACATCGGAGTCACAGCTCCCAAAAGAAGGAGAAGTC
CCGAACAATACCGAAGCCGAGGTTCCTGTCGGATATATTAGGCGGAATCTTCCGTTTGGG
CCAGATACCGGCACTACTCGACGTAAGAGATGATCAAGCCGAGTTCGTTGCTGTGTCTGA
AGCATCTTCCCTGATCATCCCAGTAGTAGCGGCTATCGATCAGAATCTCAGCCCGATCGA
GGTTGACAGTTCGATTCCTCAGAACAGGCACAGTTACACCGGTTACTACCTTCACTGCAT
ACGCCAACCGACATATGTGTACAACCAACAAACTGCTTAACAGCCAGCACTTCATTCTGC
GGAATTACACTGTCAGACGCTCATGAGAGTCCTGCAGCTGCCATATCAAGATTCAGCCGG
TACCAAGAACATAAATCGAGCTTATCGCCGAACCGATCGCAGGACAGATGTGTTGTCCTT
CATTTCTCCGTACGTACCGAGACTCCATCCAGATTATCGGAACGATACCGTAGGCGATTT
ACCAATCTCGAATATACGAGTGTCGAGTCGCGCACTACCTCCTAGAGAGGATCATATCTC
GAACAGGACACTCCTTGAGAGCCACGGAAGCTTACATCTTCGTGGTTATGATCATTCTAT
CGCGCATCACGTATTCCAAATGGTTCTTCCAGAGCTTTCGATTCTGGAGCATTTCTGTTA
AATCCAGAAGCGATACGTCGGAATCTGGGACAACTACCAACGTATGTCCTACAAGGCAGG
CCTATCATCCAAGCATCGCCGTTTATGGCAAGCTGTAGATGCCGGACAACTACGTCTGGG
ACGACTGGCGCCATCGGTTGCGTCTCGTTTAATCGGCAAGCACAAGTATCAGTATCGGCC
GTCAGGCGATTCGGGCGATCCAGTGATTGTTATCAACGCTAGTACCGTGCGATTATCAGG
TACGAAGAGGGACTGTAAGCGTTATGAGAACACTAGTTCCGAAGTCGGTACTGGAAGTCT
CCGAGAGGTTTCATTCTTATTACGACCAATCCCGTTCGCACGTTTGACATACGCGGTGCA
TGGAATGTTACCTGTTCCAGGATTGACATACTCGACCCTACAGGTCCTGCACGTGTACGA
GGGCAATTATCGCCGACTCCTAGCTGTTCAGCCAGTTCGACTTCTCCTTTAACGCTTACG
AGCAACCATTCGGTCAAAGAGGTTCCCGTTCCATATGATCTGCCCTAAGGCCTTACGGAG
AGCTGGAAGTCGTGACAATCTGCTGCGGCGGTTCCTCTTCCGACTGTTGACTGCGTTCAA
GATAGGCACTACGCATCGAAAGAGGTCTGTCAGGATCCAACAGGTAGAAGCCCTCATCAC
TAGCGCCAAGGCGAAGCCAAACGATTCGCAGCGCGTCGTTTTACATCGCCTGAGTATCAG
CATCTGTCACTCGAACCTGTTCATCCCGATCTACCATCGCTACGTTACTCGGTTCGGAGG
CTATACGCGTATTCTAAAGCATATACGGCGTCTAGGTGACATTGCTGCTGTCGCTTTCCT
GGAATTAGTGGACTAATCGACAACACAACCGGTTCATCGCGCCATTCCAAGATCCACAAT
GTCGCAAGAACCTCCAAGCCAGTACGTGAAGTCATTACGCAGGTACACAATCGCGGGAAG
TCAGGATTGCAAGATCGCTCTTGTCAGGTCACAAGATGATCTTGCCGTTGCCTCAAAGGG
TCTCAGGTACGACGGCTCTGGTCACGCGTGCAAGAAGTTCGCAAAGACCGCGTTAGAAGG
AACGGTGCAGATCGATCGACAATCCAAGGACACCTACCTGGTCCAGATCCCTACTGCGAC
AGATTTCGTAGCCCGAGTTCCACGATTCTCCAGACTGCTGTACTCCGAGTATACTGGAAA
 [Formula 2D] 
CTCCATCAGAGGCTACGCCAGATACCTTCAGAAGACTACCTTCGTCGATCGACGAAGAGC
CGTATACACAGTCCTTTCAGACTCATCAGCTGCGATTCGGGAAACAATCCGAGTCCGGCG
TATCCCTACCTCAGGACGATCAAGTTCGCTCATCATCCTACCATACACGCATATCGGTAT
CAGAATCGGAGTTTTAGTGTCTCACTGGCACTTCTCCAACTCGTACCGAAACCCTATTCT
GGCCATGCATGTCGCTGCTCGCTCACCGAGAAGCCTGAGTATCTACTTCGTTTACGGTCT
GGTCGTTTCGGAAACTTCAATTATACACTCCGTCCAGACGAAGTCTACGTCTATCCCTTG
TTACATCGGCTTCCGAACCGTTTCGGGCCGCCTGCGAAAGGCTCTATTCCCAATAGAACT
GTACGGTCAAACGTTCGTTCAGTCAGAAGTACTCGCAGTCGTCTCACAACTTAGCTTCTA
CATCAGCATCGTTAGGCAGTTCGAAGATACCAACGTGGGAGAGGGCATTAACAAGCCTTT
CGAGACTTTCCTCCGAGAAGTGACGTTCTACACGCCAAAGTAAACATTACGGAACCAAGT
ATTCCCAGGTTCGCCACCTGCAAGTATGATGAAGGTCATTTTAAATGAATACGTGCACCC
TCTGGGCAACACCGGTGACAAGCTCGAAGTAAAGCCAGGTTATGCACAGAACTTCTTGAT
TATCAAGAAGGACGCGGCGTCACATACCTTACCAAGAATTAGAACTATCCAGCCAGAGCC
AATCCAGTTCTTCCAAACGATATCTCAGAACGGCGTCAAGCCGAGTTTACCAGGATTCAA
GTTCAGGCACCGTTCCTTAGAGATCTCTCGAAAGATCGATGACACCGGCCCACTGTTCGG
CTCTATCAGTCGTCATCAAATTCTCGATCGGGTGTATCGGCCATATTCGTCGCTGTTCAA
GCAGAACATTCTATTATCGCGGTTACTTACGCGAATATCGGGACGCAAGGGTGTGTCCCT
GCAGCTAATTCTGGAGGTCAATGCCCGACTCCGTGTTCTGGTCCCGGACAATTAACCGGT
TTCAACATCATCGCAGGACGTACTGCCTTCAGCTATTATGGCACTGGAAAGTCTGGGCAG
CATCCTGTTCGTTACACATACGACATCGAACGTGGGCCGTGGTGACGGAAGCGGCGCAGG
TACCACAAGGGGTCGGGGACTCAAGGGCTCCTATGCACGGACCTCTTATCGACAGCTTAC
CCAGTTCGAGGGTGGCCCACACCCATTATTCGGACGCCTGCCGGTATTAGGTGCAGTCCC
GACTGTCTATCTGTCTACAGGACTCGTACTCGTAGTCACCGTACCGGTATCTGTTACTCG
TTCAACAGGAGTCCGTCGATCTTCGCTTCATCTACTGTCTTACATTTCACCATCTGCGTT
CGAGGTAAAGGTGCTGCTCTCATCTGGACCGAAGCTGCCACATACCACTTACAGACCTTA
CCCTGCTCGAAAGCATCAGCCGTTATCAAGTTCCAGGACAGGTACTACGAACTAAACAGC
GTTCGTTACCTACAGTGGTACTACCTTTCGCGAGACGTTTCACGTTCGATACCACACCGA
AGACATCTGGTACAGGAGAGCAAACTTCTACCAGGAGGTCCAACTTCCTGGTCTACAGCT
CATTCTCACAATCCCGTACGCATCTACGGTTCCGTACTACCGTTATTCACACCTGGTTCG
AGACAAGGCACCGTCTATACAGATTCTGCAAAGACCGGATCCGTTTCCGAGCAGTTACGC
AAATTCGTCACTTCTCCTGGCAGAATGCATTC
seqHMM5002 (SEQ ID NO: 19) [Formula 3A] 
GAATGCTCTCGGTCGACTTATCCGATATATCCCAGAACCACTCAGGTACCTGCGTCAAGT
TCGCAACTTTACAGAAAGCTCCGGCAGAGTTCCGAACAGCTCATCACAACAACGTATTCG
GTCAGGCGGTACGCTTATCTGGGTATTACAAAGCGTTCGCAGCTTCGGTCTGGAAAGCTT
TCCCATATCAAGGCGTTCCTGTCGTACCGTTACAAGCGGATTACTGCATCACCTGTCGTT
CGAACAGTCCACACACAACGGTTTCGTCTCGCAAATAATGTACCGAAAGATTAAGATCGG
CGATAGCGTCGGTGTTATCTATGGAAAGCAGAAGGGACTCGAGGGTCACGTCCACTCAGT
TCTCCGGATTCGATACCACGTGATCGTCGAAGGTGTTTTCAGGGACAAGAAGACTAA
GGCAAGACTGCTTATCCAATCAGGCGGTCCATCAGACCAGGGAGAACGTCCGATTCATAC
CTCCAATGTTAAGTCTCCGCTGTACTGGCATCATTTCAAGCATCCAGTTTTCAAGCGTGC
TCTCGAGCGATATTCAAAGAACCGGCGCAATCAAGGACAGGGCCCAGACATCTAATCTCT
TACTCGTCCGATTCTGTCCACTGGACAGCAATTACTAATGATCTGCCTGATTTCCTCGCT
CCCGATCGTATTCCACCTCAACAGGGTCAAGCAAGCCTTCGACAGAGGCCAAGGTGTGTG
CGTTACTCAGCTCATTCCAGAAGGATACAAGAAGCGCGTCCAGCAATTCCGAGGAACGGT
AATCTATCAATCCGATCGTGGTCGCAGAGGCAATTTCCGGGTGGTTAAGTCCAGTCGCGG
CCCAGGCGTCGAACGTACGTTCCGTATCTACGATCTGGTCATTGAAGGTATTATTGTGGT
CGGAACGGGCATCGTGCCTCGTGCGACACTCAGCTATTTAAGACGAGTCAGAGGCAAGGT
CGACAAGATAAAGCAATCCTTATAAACACAGGTCTTCTCAAGCCGTACTATTCGGACGAG
TTCAGAGATGATGGCACGATCAGCACGCGAACTCCGGGTGGTTACAGGCATCTCGTCAAG
AATCACTTCCAGTTTGACTGTACGGATCAGTGATCTTATCTCGAGTTCGCAGACCGTCTA
TCTGGTTGAGACGTCGTTCGACCTGGCCTCCGCCTACGTCTTCGTATCGCAGCTTATTGA
TCCAGGTATCAGTCCTGTATCAGTCCTACCACTGCTTCTCGCGAGAGGCTTCATCCGAAG
TACCCTGGAGTACTCCACGATCCTCACACTTCACCCGCCACTCGGATTCATTCGTGATTT
CAGCCTGAGACGTTCGTACCGAATCCGAAGTCTGATTCTTCAGCTGTTCGAGCCTTAAAC
TGCCAACGACTTCGATTCACCTTACGATACGGCCTATACTATGGTCGAGTACTTCTACGG
ATACCCGCTGACGGACGGCCCAGGTCAGACGCTCGGCCCTATTCTTCGCCGTGTCCTCTT
AGGTCCAATTGCAGGTTCTGCGATCTCGAGGACTCTAATTCGCCCACAAGAAATAGAATT
CCTCAATCGAGCAGGAGTGCCTGAAGATGTGAACTATATCATTAGGAACTTGAAGAGCTT
GGTTCGGCATATTACTTCATCGAAGTCAGCCGGTATCTCGCTGGTACGTTTCGATTTATT
CGACGGTCGGAGGGCTCAACGCAGCTGGTATTCAAGACAAGACGTCGAAGTCCGTTTCAG
CCCTCACCCAATTAATCATTTATTACGTCGGGAAACTTTGGATATTAGCCTGATCGTTTA
CTGTGGCCCGGGCTACCACCCTTTCGGATCCACATACAATCATCTGCGCGCAACTTTCGG
CATTACAGTGGATGCACGATTCACCCCTGTTAGCTTCGTGCACTATACGGTTCATTACGA
ATTCTCCCAAGGAGCTTCTCCGGAAGATGCACTGTACCTGGATATCCGAACTTCCTATCG
CCGAAGGACATATCCTGCTTTGTACTGTGCGTCACCGTCCCTGGAACAGATCCTGTCGGG
ATATCATACACGAGAGCGCACGTACAGATCCTATACGCCTACTATTCGAGCCACTACGGT
CAACCGAGAGCAAATTCAGTCGAACAGACAAGACATCACCGACCTTGATTTGTCATCGCG
CTCGCTGAATTGCCTGCGATCCTTCGTCATACCAATCCTGCACGAACTGGTGTCTGCAGG
ATACGCCGAACTAATACCAACCGGCAACTTGGGCGACAAGTCAGCCTGGGAAATCTCGTT
CCGTCTCACTGTACGTTTCCTGGCGTTAATCGTATCATAACAGCAGGTCATACGTACTCT
GCAAGGAAGTCTCTTCCCTGCAATGTTCGACGATTTCTTACCTGCGACTGTGAGTTCCGG
CCGAGGTGGAGTCGGTTCTCGCTTACCAAGGATCGAACGATATTCGACAAACGGTGGACC
GCCGGGCGGTCTAGGTGGCCCTGGCGGTTCGGTGATTCGAGGTATCTGCGCAGTTACTTC
GCATCTTTCTGTTCAATCGCAACTCAGGTCAGCATACGCAACACCGGGCAGCGAAGGTCC
TCAAGAAGAGCCAGTAGGTCGTCGCAGAACATCAGAGCCGATCATAGTCCCGCCTGGCCG
ACTAGTTACAGCTTCTGCGCACTATCATACCGACAACAGGGTTTCTTCAGCACGTTCACG
 [Formula 3B] 
CCATTATACAGGTCAGGGCGGCTACAGAGGAGATGGTAACAGGTCATTCAAGAAGCTGGA
AGACCCTCTTCCGCTGTCACGCACTTCTGGCAGAGGTACTACGTCCCAAGGACTGTTCTT
ACCATTATCTTACTTGGCCGATGTTGGTCTCGTTGGTCTCGTACAAGACGGCAAGAGCGC
ACTGTTGCTCACAAGAGCTGTATCGACACGAACAATTAAGCTATACGTCTTCATAACGCA
CTACCCGACCCTGGGCTCAACCTGCGGAGAACCTTATACTCCGTCCTCTCAAGCTGATAT
TCCGGGCTTAATCCATCTCAGACACACGGGATCAGGCCTGGGTGTCAAGTTCTTGACCCA
TATCAAGATTCGAGAGAGTCTGCAAGTACTGATCGAGAGTCTTCCTGCATTACCAGACGT
CAGACTATCTCTGCACCAGAAGGACTCAGTTTCGTTAGTATCCTATTTACGTCTCCTGAT
TCCTTCGATCGACTGTACTCTGGCCAGAAAGTACGATTTCACCGTCTTCCAACACGTACG
GTCTCAGCTGCTTCTATCACGGTCATTACAGCGAACATTCCTCCGATCGGCTACATATCG
CTTCCAGTTCACCCTGTTCCATCTGGCGGTACTACAACCGCTTTCCGATTACTAACCTGT
TCACTCCTCCGTTCATACACGTCCAAAGTTCCAGGAAATGCGAACTGTAGCGATCCTGGG
CGCCTTCAACTCCGGCAAGAGTACTCTTCTCAACTTATTATTACGTACTGTACCGAGGAT
CACATCTTCTCAGCATCTAACGACCCTACAATCTATCCGAGGTACTCGAGGTCATACGAT
ACATCAGAGGATTTTCATCGATACACCGGGCTTATACAGAAGGAAGCTTCGATCTGCGGA
GCTGCTGGCAAAGTACATCATACGGGCGCTGATCACGGTGGATCTGATCCTTTTCGTTGT
CGCCTACGCTATTCGTCAACCGAGAGATCACTCTCTGACAACTCGACTGTCTTTACATCG
CACTTCGAGGAGACTGTCGTCACCAACCCATATTCGCACTTCGAGTCCACTGTTCTCAGA
CTACACTCCTCGTTACCTACGAGCATCCTCGAAGATACGAATTGTGCAGATTTGCACAGA
TCTCGGTTCCAGCAATTCACTTACGCATCAGACGAGGAGACTCAAGCTACAACCTTTCAT
ACGCAGGAACCCTTCAGATGCTGTGTCGGACCGTATTCTACATTTCGGTATCAAGGAAGT
TATCCGTGAAAAGATTCTGTCAGCCACCCAGAACGAGATCCCGTACGCTGTCCCTGTGTC
CATCAGATCCGAAATCTCGCCAGAGGATTTCGGTATACTCATCAGATCGAGCATTAGAGT
GCTGCGCATCTCACAAAAGTTAATAATCATCGGCAAGGCAGGTGATCACATTAAGTCAAT
TGGCCCTCATCGTCGCAGCGTCCTAATTACTCGGATCTGCTCCTACGTAATTCTCTTCTT
AACAGTGAAGTAAGAAGGACCGTTCCTTCTTATTCGCGGACATCGATTCACACCTATGGG
ACACCCGAAGACTCAGGGACACTCTAAGTCTTCGGACGCACGACTTTCGGGTTCCAGACG
AGGTCTTACTTTAGTGTCCTTCGGTAAGTACTTCTTAGCCATTCATCGATCTACCTACAT
TCGTCAAGCTCCTATTCACCGACGTATCGAGGCGTTACGACGGGAGGTCCAGTTCATCGG
AGACCTGTGGATACGCATAACGTCTCTGCCGGTATACCACCTCAAGCCTGAATTATTCCG
GATGGGAGACGGCAAGGGTCGTTCGGAAACATGGATTACATCAGTTCATCAGGGCTTCGT
GTTGTTCGAATTGTCTGGCGTTGGACTCAGAGGCATCGACAACGCTTTGGATCATGCATA
CGTCAAGCTGCCAACATAAACTATCCCAGGTTTCATTACTTCCAAGACGGACCTACCAGG
CATGGTCCTGTTAGTCGCGGTTAGCGGCGGACCAGACAGTCACCAGCTCCTGATTCATCT
GGCACGTCTCCGACTTCAAGACCTGCATCGTACTCAGGCCGCACTGGTCGATCATACATA
CCGGACAGCATCCTATATCCCAAGCCGAATCGTCTATCGTTCACTCGGAGTCCATTCAAC
TGCAACCCTCAACGATTCCTCACGAAAGGGATCCTTCCGTCCAATCTGGGTACGAAACGG
TGAAATCACCGCGCGCTCTCAGCGTTATAACTTCCTCACTACTACGACAATCAGGGAGAA
CCTCTCGTACATTTTGACCGCGCACCACTGTGATGATGTAATCGAAACGATTTTGAGACA
ACTGCTTCGCGGTCTATCTACCCAGGGCCTGATAGGCTTGCGCAATCCTACGTTCAACTG
CAGAGGAGCAAATATCTCGCGCCCGCTGCTGATCTCAGAAAAGCTCGAAATTCCATCTGC
AGGTTCAACAACCAGTTTAAAGCAGTACCACGATTACACGAATTCATCCGACTCTTACTC
TCGTAATACTATTCGTCATCACTTGCTGCCTTAAAGGTATTCGTACGAACTGCCTCGTTC
GCCTATTCAAGCCTCTATGATTAATAGCAAGATACTCAACGGTGAAGTCTTCCTGATTTT
ATCGATCGGTGGTACCTCGCTCATTACTCTCTACGTTGAGGCCCTGGCAAGCGCCAGCAA
 [Formula 3C] 
TCCTGTACTGGACTTGGTTGTAATTTCAGTTCGTACCCATCTGCCGGTAGTTAAGATTCT
TGATTATGGTAAGTACTCGTATGTATCCTACAAGGAGACTAAGTACCGGAAGAAGACGCA
GCCTCAAATTTGGGTCAAGGAAGTTGAACTCTCTGTTCACATTCAAACTCCTGATTATTC
AGCAAAGCGAGCTCACTGTCGCAACTTCCTGACCTACGGTTCGACGGTGAAGCCTCTAGT
ACAACGGTCAGGCCGATTCAATCGCCAACGATCGTTATCGCACTCTGTGCTTACGTACGT
GTATCGTCCAAACGGTACCGTCATCATCCAGCATCAAGAGTTCCCACAGAGTCCGTTCTC
ACAGCTACCGAACCTGCTGATTCCAAGAGGCCCTTAACACGAATCGGATCCTGTCTTCTG
TACTACACGGTCGCTTATCATGCCGGTCATCTTATTCACTCTTCGGAGGTCCATCATCCT
GTCAGGCGATTCCCAGCATACCTGCAACCCGCAGTTCACTCAGTTCATCCTGCTGCACCG
TCATCAACACAGCCTTATCCCACTGGCGTTCCAGTCACCTTGCCTTCTTACGGTACAAGC
TTCGGTCCGAACCTGCTCCGACTACTCATTCGACATACTGTTCGTGGGCACAAAGAACCT
CTGGTCGATATTCGTCTCTCCGCTTGCTAATTCCACAGGTCTCTGCTACGTGTCTCAGCG
TTGGTTGGGTGGCCTACTGACCAATACTACTACCATAACCAGCGGCATTTCAACGCTGCC
GGATCTGTTATCGCTCACCTGGCACAAGTCACAGAGACGCTCGATCAAGAAGGAAAGCGT
CAGAGTTACCATCGAAAGAGCGATTTTATTCTCCTACCTGGCTGGAATTGCCAAGCTGCA
AGACGCACCGACGTTCCTCACCGTGTTCGACCAAGCACTTGAAGTATCAGCCGTGACAGA
GGCGTATCGCCTGTCGATCCCGGTTGTTGCCGGATTCGATGTTAATTTCGGCCCGAGACA
TGTTGATGCGTCCATCCCAAGTAATATCCACGAACGCAGTTCTTATACGTTAATCGCGTA
TACATCTGGCACATCGGTTGCAACAATATCCGCGTCCTAATACTCTACAGCTGGCTTCCC
AACGAAGTACGTCCGTCCATTCATGTTACACCTGCCGCTGTCGAAGGAAGATCCAGTCAT
CGAACAACATCCTAACCCGAAGTACCGTTCGTCAGACTCCTTAACCGACGTACTGAGTTT
CTCAAAGCTGCTTCAATATCGAACCGGTCATACGTCGAGCCAGCTTCAGTTCGGCGACCT
CACGATATCTAATCTGGAAGTGTCACCGGATGCGCAATCAGCCACCTTCGTTTACCTGGA
CGGCAAGCAATTCCTGTCAGGACATGGCCGCCTTCATTTGTCAGGTTATGATCATCAGGT
CCATCGTTCCATTACGCCAATGCGCGCTATAGAATATCGGATTCTGCAGAGATCGTCGTA
ATCGCGCAACATCAGTCAGGTCTCATCAATCTACGCACGATCTATGTACACTCGAAGACA
ACAGCTGTACGATACGGACCGCACTTGGAGACGAGTGGATGCCTCAAGACGCATACTGGG
CCGCCTTGCTTCGCTCGTTGCAATCGTATTGTACGGAAAGCGAAAGGTACTCTACCTCCC
GGGTCTCGACTCTGGCGATCAGGTTATCGTAATTAATGCTGACCTGGTACCACTGTACGG
CCATAAGAACACGCCTAAGCGCTACACAGGCTATTCGCAGTTCATTGGTCAGCGTTACAC
CTCGCTGCTGTTCAAGCTCGAATTCCTCCAACCGGAGAGTTACCTCTCCGTTGCCGTAGT
TGGAGTGCTCCCGCCTACCATACTGGCCGCATCAAATCATCTCGAACTGCGTGTTTACCA
AGGCACTCCGACAAGAGACATCGCGAGTCTCCCGTACAATACCTGGGATTAAACATTCTC
GAACAGTTCTGCGTCAATATCCCAAGGCGCTCTTATGAGATCACAAAAGGCGGTTCGTTA
CCTCCGACTCCGTGGTACATTACTCCGCTTCCCTTATTTCGAGCTGCTGGAGCATCGACG
AATCAGCACCACGATACTAAAGCTACTGCACCACTTCGTTCCTGTAGAATTCTTGATTAC
CGAAGCAAAGGCTATACTCGTCTCAAACCAGCGCCCTGTGTTCGTACGCCTGTTCAAGAG
CACTTCTCTACGCTCTTTATTCGTTCCAATTGTCATTCCTTACTTACCTCGTTACGGTGG
CTATACCCGGATTCTCAAGCCAAGGTCGCGCAGAGGCGATGGAGCTTCCTACGCCGTCCT
GGAACTGGTGGACTAATCACGCAAGTACGACAGCTTCACAGGCTACCGAATACTTCCTAT
GAAGCGACACTTCGTATCAAGGCCTGTCAAGAATCTTCGTGATCTAACCTTCGCGACCTT
CCAGGATTGCAAGTCGGCGCTGTCCCTCGTACATTCGGACCTGAGCAGCGCCTTCGGCAC
GCTGCGTATTCGCGGCTACGGTTCCGCGTACAAGAAGGCAGTCCCGATTGCAAAGGAAGG
CTCTGTGCTACTGTACAACGCACTGCTTTCTCCGAAGCTGGTGGACATAGATTACGAGAC
CGATTTCGTCGCCACAGATTCCTCTTTCAGGGATCTGGTTTATCGAGAGCGACGTATTCT
 [Formula 3D] 
CAAGCTGTCCGAAGGTTCTGTTCCAAAGCCGTCGTTATATCCAGCACGAGCCATCAATCG
AGGAAACGAACCGTTGATTCTGCTCCTCGCAACTATATACGAGACCATCCAAGTGCGGCG
GATCCTGGCCTCTTACATTTCCTGCCCAGCTTCTATTTACCTTTATCTCCACAGTTTCAT
ACGTATCGGTGTATTAGTATTCCGTCGCGATCCTCTCTCTACACGCGATCTCCATATTCT
TGCTATGCATGTGGCGGCTCAATCCCCGCGTTCACTGATCAAGCTTTCGGTTCACCGGCT
GTTAGTATGCCGTTATTTCAGAATCATTTCACCGCCAGCCTATCCGAAGTCCTCACCGTT
CGATATTACTCGACCGAACGTCGTCGGACGGTTGTTCAAGACAAGATTCCCAATTGCTCT
GCCTGTCCAGACTTTCGTGATATCCTTCGTTACGGCTGTTGAGCACATCCTCTTACGATA
CCTTCACGCAGTACTCACTTTCTACATCGGTATCGTGGGCGAAGGTATCCTAAAGAGTAT
CGACAGGTTCATCCTAGAAGTGCCGTACCACGCATTACAATAACGACAACACGAGTCGTT
ACACCAGACCAGTCTTACCACAGATATGATGAAGGTTATTTTGAGGGAATCCGTTTCAAC
ACTGGGCACGCATGGTGACACCAACGAAGTTAAGAACGGCTACGCATACAACTTCCTCAT
CCCGAAGTCTCAAGCGTACGCCTATACGCAGATCACAATCGGACATCACCCAGAATCTGT
CCGATTCCTCAACGCAAGACTCTCCCAGTACACAACATCAGCCATTCTGCCACTGTACGC
AAGTCGAACGAGACTACTGCGCATTTCTGTCAAGACGGGAGACCCTGGTGTCCTCTTCGG
AAACATTCATCCGATTCGAATTCTCGACGTAGTGAGAACGAACGGTTCGATCCTGTCTAA
GCAACCGATTTACCTGCAAAGGAACTGGCTCAGCTTCCGCGGAGAGGAGGACGTTTACCT
GTCGTTACTGCTGGAGGTGTCAGCGGGTTTGTTCGTGACGGTTATTCTGGTCTAATCACG
TCAATCCTCTCGACAATACGAGATTCAGACGCGCATTATGACTTTATCCTCGCTACAATC
TGTCCCAGAAAGCCCTTCCGACAGACAATCTGTCGGACGCGGACCAGGTTCAGGCCTCGG
TCACACGCATGGCACGGGCAAGAAGGGTCAGGCTGCTCGCGCAAGAGAACCTTCTCCAAG
ATACTTCGAAGGCGGTCACTGCCCGTTAGATTCCCGCCTGCCTACGCAGGGCTCCGCCGA
TTCGTCTTCTTACGCTATATACTCGGTGGCTGTGTCTCCGAGCAAGTTCCTACGCTATTT
CAGAGCTGGAGTGCGGCAAGCTCCACTCAAGAAGCTGTATCAAATTTCTGCTACGCCAGT
CCCAGTAAAGGTGTTAAAGTACTTACCTACACCTGCAACCGGTTCTGGTATCGTTTACAG
ACAATACCCTACCCAGCACCTGTCTGGCACAGTTATATTCGACTTCAGCACGTAAAACGA
AGCGTCGGCAATTTACTGTTCAACTCACCCGCATTACTGTACCTGTCCTTCTGTCAAGAG
GGTACAAGTCGGTCCTACTTCGCAAACTCAGCAAAGGGTTTCTCACGAAGACCCATCCGT
CACATATCTGGTATCCTGTCAGACGAAGCAGAGGTTCGAAGCGATTTCAAGGTCTACTCG
ACAGAGGTCCTCCACTTATCCGTACTTCACAAGCGTCAGTCCTACACTCCGTCACTACAG
ATACCTATCTCAGCTCACTCGGGTATGCATTC
seqHMM5003 (SEQ ID NO: 20) [Formula 4A] 
GAATGCACACCTTCGCATCAAGACCTGCGAACTACAGATACCACCTTACAGCTGGATTCC
AAGACCGGGAAATCTGGAATCCTTCGGTTCTTACCGGACAATCGAGCTCAAAGAACCGCA
GTCAACTCTTACATCGGGAGGACATCAGTCTTACACGGCTACACGTTTACAGCAGACATC
CTGGTCCTTCGATACGCCTATCTGCTGTTCAAATCGCACGTCGCATCACATCGAGAGTCG
TCACTTTCAACTACGGAGTCCTCAACCTGTCCAAACTATGCACTATAAGATTAAGAGCGG
AGACAGAGTGACAGTGATCCCTGGCAAGTCCAAGGGCTCCTTCGGATACGTGAGCTTCGT
TACAAAGGGATCTGGAACGGTGATTGTGGAAGGCGTGCGGAATTCTAAGAAGCGGGAAAA
GTATCCAACTACGGAGACACATCTGGGTCTTCACCGTCTCAAGGAAAGGCCTATCCCTGT
TAGTAACGTTGCTGGCCTTCGGTACACCTGTCACACAAGCATCTCAGTTACTGGCCGCAT
CCACGTTCTGTCCAGCAAGTCACGGAATCTCGGACGAACTGGTCAGTTCATATAATCCAA
GTATTACACCTTCCGCCAATCTGTCAAGATCGTCCCTATGAAGGAACAGATTCCTCGATT
GATCCGACATCTTCGGATACGCAGAATACCGAGCCTTTTCAGCGCCGGTTACAGGGTAAC
TGTACAACAGGTCATTTCGGAAGGTCACGTATCCCGTTACCAAATTTTCGTCGGTCGTGT
TATCTCTGCCACCCTTCAAGGCGAATTCAGCCCTGCTCAAGTAACCAAGTACGCATATGG
CCCGGGCGTAGAACGTATCTTCAGCTGGACACGTACTGCAATTCATACAATCGGCGTCTC
GTTCACTGGCATCGTAGAGCGTGCAGGTCTCACCTATCTTCGCGTTACAACAGGCAAGTT
CTCTAAGATTAAGGGTTCATCGTAACTATCGTACCGGCTTCAAGATCGATCGCGTATTCC
TCGCATTATGATGGCGGCTGTATACCGAAACACGCGTGTACATCAATCGATCACTGCGAA
TATCCTTCGCCTGCTCCAGTATACCATTGACGACGGTACATTCCTCTGTCCACCTGTTCA
TATAGTGGAGGATTCAGAGGATCTGCTTTCCGCGCTTGTCTTCGTGGCTACGTTAAGCGA
TTATACAAGGTCGGCTATACAGGTTCGAAGCCTTCCAAGTGCCATTGGATTCATTCAGTA
CAGATTGCTGCGGTACGCTTCACTGAGAGCTCATCCGACTCTGAACTTCTCGCCTGATGC
GAGTTTGTGTACAGGAGAACGGATCGAAGCTCTGATTCACGCATTGCACAATCGGTAACG
CACAGATACAGGACACTTCCCTTACGATACGGCCTATACTATGGTTGAGTACTTCTACGG
ATACCCGCTGACCGACGGCCCAGGTCAGACGCTCGGCCCGATTCTTCGCCGTGTCCTCTT
AGGTCCTATTGCAGGTTCTGCGATCTCGAGGACCCTAATTCGCCCACAAGAAATAGAATT
TCTCAATCGAGCAGGAGTGCCTGAAGACGTGAACTATATCATTAGGAACTTGAAAAGCTT
GGTTCGGCATATTACTTCAAGTAAGTCAGCCGGTATCTCGCTGGTACGGTTCGATTTATT
CGACGGTCGGAGGGCACAACGCAGCTGGTATTCAAGACAAGATGTCGAAGTCCGTTTCAG
CCCTCACCCGATTAATCATTTATTACGTCGGGAAACATTGGATATTAGCCTGATCGTTTA
CTGCGGCCCGGGCTACCACCCTTTCGGAAGCACATACAATCATCTGCGCGCAACTTTTGG
CATTACAGTGGATGCACGATTCACTCCTGTTAGCTTCGTGCACTATACGGTCCATTACGA
ATTCTCCCAAGGAGCTTCCCCGGAAGATGCACTGTACCTGGATATACGAACTTCCTATCG
CCGAAGGACATACCCTGCTTTGTACTGTGCGTCACCGTCGCTGGAACAGATCCTGTCGGG
ATATCACACACGAGAGCGCACGTACAGATCCTACACGCCTACTATTCGAGCCACTACGGT
GAACCGAGAGCAAATTCAGTCGAACCGACAAGACATCACCGACCTTGATTTGTCTTCGCG
CTCGCTGAATTGCCTGCGATCGTTCGTCATACCAATCCTGCACGAACTCGTGTCTGCAGG
ATACGCCGAACTAATTCCAACCGGCAACTTGGGCGACAAGTCTGCCTGGGAAATCTCGTT
CCGTCTCACGGTACGTTTCCTGGCGTTAATCGTAAGCTAACAGCAGGTCATACGTACTCT
GCAGGGAAGTCTCTTCCCTGCAATGTTCGATGATTTCTTACCTGCGACTGTGAGTAGCGG
CCGAGGTGGAGTCGGTTCTCGCCTCCCAAGGATCGAACGATATTCGACAAATGGTGGACC
GCCGGGCGGTCTAGGTGGGCCTGGCGGTTCGGTGATTCGAGGTATTTGCGCAGTTACTTC
GCATCTTTCTGTGCAATCGCAACTCAGGTCAGCATACGCCACACCGGGCAGCGAAGGTCC
TCAAGAGGAGCCAGTAGGTCGTCGCAGAACATCCGAGCCGATCATAGTCCCGCCTGGCCG
GCTAGTTACAGCTTCTGCGCACTATCACACCGACAACAGGGTTTCTTCAGCAAGATCACG
 [Formula 4B] 
CCATTATACAGGTCAGGGCGGATACAGAGGAGATGGTAACAGGTCATTTAAGAAGCTGGA
AGACCCTCTTCCGCTATCACGCACTTCTGGCAGAGGTACTACTTCCCAAGGACTGTTCTT
ACCATTATCGTACTTGGCCGATGTTGGTCTCGTTGGCCTCGTACAAGACGGCAAGAGCGC
ACTCTTGCTCACAAGAGCTGTATCGACAAGAACAATTAAGCTATACGTCTTCATAACCCA
CTACCCGACCCTGGGCTCAACCTGTGGAGAACCTTATACTCCGTCCTCTCAGGCTGATAT
TCCGGGCTTAATCCATCTTAGACACACGGGATCAGGCCTGGGTGTGAAGTTCTTGACCCA
TATCAAGATTAGAGAGAGTCTGCAAGTACTGATCGAGTCACTTCCTGCATTACCAGACGT
CAGACTCTCTCTGCACCAGAAGGACTCAGTTTCTTTAGTATCCTATTTACGTCTCCTGAT
CCCTTCGATCGACTGTACTCTGGCCCGAAAGTACGATTTCACCGTCTTCCAACATGTACG
GTCTCAGCTGCTTCTATCACGCTCATTACAGCGAACATTCCTCCGATCCGCTACATATCG
CTTCCAGTTCACCTTGTTCCATCTGGCGGTACTACAACCGCTATCCGATTACTAACCTGT
TCACTCCAGCGTTCATACACGTCCAAAGTTCCAGGCAATGCGAACTGTAGCGATCCTGGG
CGCCTTTAACTCCGGCAAGAGTACTCTTCTCAACTTATTATTACGTACTGTACCGCGGAT
CACATCTTCTCAGCATCTAACGACCCTACAATCTATCCGAGGTACTCGCGGTCATACGAT
ACATCAGAGGATTTTCATCGATACACCGGGCTTATACCGAAGGAAGCTTCGATCTGCGGA
GCTGCTGGCAAAGTACATCATACGGGCGCTAATCACGGTGGATCTGATCCTTTTCGTTGT
CGCCTACGCTATTCGTCAACCCAGAGATCACTCTCTGACAACTCGACTGTCTTTACATCG
CACTTCGAGGCGTCTGTCGTCACCAACCCATATTCGCACTTCGAGTCCACTGTTCTCAGA
CTATACTCCTCGTTACCTACGAGCATCCTCGAAGATACGAATTGTGCAGATTTGTACAGA
TCTCGGTTCCAGCAATTCACTTACGCATCAGACGAGGAGACTCAAACTACAACCTTTCAT
ACGCAGGAACCCTTCAGATGCTGTGTCGGACCGTATCCTACATTTCGGTATCAAGGAAGT
TATCCGTGAAAAGATTCTGTCAGCCACGCAGAACGAGATCCCGTACGCTGTCCCTGTGTC
CATCAGATCCGAAATCTCCCCAGAGGATTTCGGTATACTCATCAGATCGAGCATTAGAGT
GCTGCGCATATCACAAAAGTTAATAATCATCGGCAAGGCAGGTGATCACATTAAGTCAAT
AGGCCCTCATCGTCGCAGCGTCCTAATTACTCGGATCTGCTCCTACGTAATACTCTTCTT
AACAGTGAAGTAAGAAGGACCGTTCCTTCTTATTCGCGGACACCGATTCACACCTATGGG
ACACCCGAAGACTCAGGGACACTCTAAGTCTTCTGACGCACGACTTTCGGGTTCCAGACG
AGGTCTTACTTTAGTGTCCTTCGGCAAGTACTTCTTAGCCATTCATCGATCTACCTACAT
TCGTCAAGCTCCTATACACCGACGTATCGAGGCGTTACGACGGGAGGTCCAGTTCATCGG
AGACCTTTGGATACGCATAACGTCTCTGCCGGTATACCACCTCAAGCCTGAATTATTTCG
GATGGGAGACGGCAAGGGTCGTTCGGAAACATGGATTACATCAGTTCACCAGGGCTTCGT
GTTGTTCGAATTGTCTGGCGTTGGACTCAGAGGCATCGATAACGCTTTGGATCATGCATA
CGTCAAGCTGCCAACATAAACTATCCCAGGATTCATTACTTCCAAGACGGACCTACCAGG
CATGGTCCTGTTAGTCGCGGTGAGCGGCGGACCAGACAGTCACCAGCTCCTGATTCATCT
GGCACGTCTCCGTCTTCAAGACCTGCATCGTACTCAGGCCGCACTGGTCGATCATACATA
CCGAACAGCATCCTATATCCCAAGCCGAATCGTCTATCGTTCACTCGGAGTCCACTCAAC
TGCAACCCTCAACGATTCCTCACGAAAGGGATCCTTCCGTCCAATTTGGGTACGAAACGG
TGAAATCACCGCGCGCTCTCAGCGTTATAACTTCCTAACTACTACGACAATCAGGGAGAA
CCTCTCGTACATTTTGACCGCGCACCATTGTGATGATGTAATCGAAACGATTTTGAGACA
ACTGCTTCGCGGTCTAAGCACCCAGGGCCTGATAGGCTTGCGCAATCCTACGTTCAACTG
CAGAGGAGCCAATATCTCGCGCCCGCTGCTGATCTCAGAAAAGCTCGAAATTCCATCTGC
GGGTTCAACAACCAGTTTAAAGCAGTACCACGATTACACGAATTCATCCGATTCTTACTC
TCGTAATACTATTCGTCATCACTTGCTGCCTTAAAGGTATAGTTACGAACTGCCTCGTTC
GCCTATTCAAGCCTCTATGATTAATAGCAAGATACTCAACGGTGAAGTCTTCCTGATTTT
ATCGATCGGTGGTACCTCGCTCATTACTCTCTACGTCGAGGCCCTGGCAAGCGCCAGCAA
 [Formula 4C] 
TCCTGTACTGGACTTGGTTGTAATTTCAGTTCGTACCCATCTGCCGGTAGTTAAGATTCT
TGATTATGGTAAGTACAGCTATGTATCCTACAAGGAGACTAAGTACCGGAAGAAGACGCA
GCCTCAAATTTGGGTCAAGGAAGTTGAACTCTCTGTTCACATTCAAACTCCTGATTATAG
CGCAAAGCGAGCTCACTGTCGCAACTTCCTGACCTACGGTTCGACGGTGAAGCCTCTAGT
ACAACGGTCAGGCCGATTCAATCGCCAACGATCGTTATCGCTTTCTGTGCTTACGTACGT
GTATCGTCCAAACGGTACCGTCATCATCCAGCATCAAGAGTTCCCACAGAGTCCGTTCTC
ACAGCTACCGAACCTGCTGATTCCTAGAGGCCCTTAACACGAATCGGATCCTGTCTTCTG
TACTACACGGTCGCTTATCATGCCGGTCATCTTATTCACTCTTCGGAGGTCCATCATCCT
GTCAGGAGATTCCCAGCATACCTGCAACCCGCAGTTCACTCAGTTCATCCTGCTGCACCG
TCATCAACACAGCCTTATCCCACTGGCGTTCCAGTCACCTTGCCTTCTGACGGTACAAGC
TTCGGTCCGAACCTGCTCCGACTACTCATTCGACATACTGTTCGTGGGCACAAAGAACCT
CTGGTCGATATTCGTCTCTCCGCTTGCTAACTCCACAGGTCTCTGCTACGTGTCTCAGCG
TTGGTTGGGTGGCCTACTGACCAATACTACTACCATAACCAGCGGCATTTCAACGCTGCC
GGATCTGTTATCCCTCACCTGGCACAAGTCACAGAGACGCTCGATCAAGAAGGAAAGCGT
CAGAGTTACCATCGAAAGAGCGATTTTATTCTCCTACCTGGCTGGAATTGCCAAACTGCA
AGACGCACCGACGTTCCTCACCGTGTTCGACCAAGCACTTGAAGTATCAGCCGTGACAGA
GGCGTATCGCCTGTCGATCCCGGTTGTTGCCGGATTTGATGTTAATTTCGGCCCGAGACA
TGTTGATGCGTCCATCCCAAGTAATATCCACGAACGCAGTTCTTATACGTTAATCGCGTA
TACATCTGGCACATCGGTCGCAACAATATCCGCGTCCTAATACTCTACAGCTGGCTTCCC
AACGAAGTACGTCCGTCCATTCATGTTACACCTGCCGCTGTCGAAGGAAGATCCAGTCAT
AGAACAACATCCTAACCCGAAGTACCGTTCGTCAGACTCCTTAACCGACGTACTGAGTTT
CTCAAAGCTGCTTCAATATCGAACCGGTCATACGTCGAGCCAACTTCAGTTCGGCGACCT
CACGATATCTAATCTGGAAGTGTCACCGGATGCGCAATCAGCCACCTTCGTTTACCTGGA
CGGCAAGCAATTCCTGTCAGGACACGGCCGCCTTCATTTGTCAGGTTATGATCATCAGGT
CCATCGTTCCATTACGCCAATGCGCGCTATAGAATATCGGATTCTGCAGAGATCGTCGTA
ATCGCGAAACATCAGTCAGGTCTCATCAATCTACGCACGATCTATGTACACTCGAAGACA
ACAGCTGTACGATACGGACCGCACTTGGAGACGAGTGGATGCCTCAAGACGCATACTGGG
CCGCCTTGCTTCGCTCGTTGCAATCGTATTGTACGGAAAGCGAAAGGTACTCTACCTCCC
GGGTCTCGACTCTGGCGATCAGGTTATCGTAATTAATGCTGATCTGGTACCACTGTACGG
CCATAAGAACACGCCTAAGCGCTACACAGGCTATTCGCAGTTCATTGGTCAGCGTTACAC
CTCGCTGCTGTTCAAGCTCGAATTCCTCCAACCGGAGAGTTACCTCTCCGTTGCCGTAGT
TGGAGTGCTCCCGCCTACCATACTGGCCGCATCAAATCATCTCGAACTGCGTGTTTACCA
AGGTACTCCGACAAGAGACATCGCGAGTCTCCCGTACAATACCTGGGATTAAACATTCTC
GAACAGTTCTGCGTCAATATCCCAAGGCGCTCTTATGAGATCACAAAAGGCGGTTCGTTA
CCTCCGACTCCGTGGTACATTACTCCGCTTCCCTTATTTCGAGCTGCTGGAGCATCGACG
AATCAGCACCACGATACTAAAGTTGCTGCACCACTTCGTTCCTGTAGAATTCTTGATTAC
CGAAGCAAAGGCTATACTCGTCTCAAACCAGCGCCCTGTGTTCGTACGCCTGTTCAAGAG
CACTTCTCTACGCTCTTTATTCGTTCCAATTGTCATTCCTTACTTACCTCGTTACGGTGG
CTATACCCGGATTCTCAAGCCAAGGTCGCGCAGAGGCGATGGAGCGTCCTACGCCGTCCT
GGAACTGGTGGACTAATCACGCAAGTACGACAGCTTCACAGGCTACCGAATACTTCCTAT
GAAGCGACACTTCGTATCAAGGCCTGTCAAGAATCTTCGTGATCTAACCTTCGCGACCTT
CCAGGATTGCAAGTCGGCGCTGTCCCTCGTACATTCGGACCTGAGCAGCGCCTTCGGCAC
GCTGCGAATTCGCGGCTACGGTTCCGCGTACAAGAAGGCAGTCCCGATTGCAAAGGAAGG
CTCTGTGCTACTGTACAACGCACTGCTTTCTCCGAAGCTGGTGGACATAGATTACGAGAC
CGATTTCGTCGCCACAGATTCCTCTTTCAGGGATCTGGTTTATCGAGAGCGACGTATTCT
 [Formula 4D] 
CAAGCTGTCCGAAGGTTCTGTTCCAAAACCGTCGTTATATCCAGCACGAGCCATCAATCG
AGGAAACGAACCGTTGATTCTGCTCCTCGCAACTATATACGAGACCATCCAAGTGCGGCG
GATCCTGGCCTCTTACATTTCCTGCCCAGCTTCTATTTACCTTTATCTCCACAGTTTCAT
ACGTATCGGTGTATTAGTATTCCGTCGCGATCCTCTCTCTACACGCGACCTCCATATTCT
TGCTATGCATGTGGCGGCTCAATCCCCGCGTTCACTGATCAAGCGTTCGGTTCACCGGCT
GTTAGTATGCCGTTATTTCAGAATCATTTCACCGCCAGCCTATCCGAAGTCCTCACCGTT
CGATATTACTCGACCGAACGTCGTCGGACGGTTGTTCAAGACAAGATTCCCAATTGCTCT
GCCTGTCCAAACTTTCGTGATATCCTTCGTTACGGCTGTTGAGCACATCCTCTTACGATA
CCTTCACGCAGTACTCACTTTCTACATCGGTATCGTGGGCGAAGGTATCCTAAAGAGTAT
CGACAGGTTCATCCTAGAAGTGCCGTACCACGCATTACAATAACGACAACACGAGTCGTT
ACACTGCCGAAGAATCTCACCACGTATGATGAAGGTCATCCTGTCTGAAATCGTACAGGG
TCTGGGTTCAACGGGAGATCGGGTCGAAGTCAAGTACGGCTATGCGTACAATTTCTTAAT
TCAGAAGTCTTCTGCCCCAGCGACTACGTTACTGGCAATTCCTTCTCAACGTCGAAGCTC
GTACACAACATTCCAGCGTCAATCTCTCGCATCAAGCATCGCTGTTATCAACGCGTCGGG
AATACAACAGCCGAATTTGTCGATTGACGATAAGGACCGGGACTCAGGACACCTATTCGG
CAGCATCCGTCGAGCAGAGATCTTCGATCGAGTCTATCGGTTCCATACACCATTATGGAA
GGGAATTATTTCGTTACAGACATCGATACGAGACACCAACGGTGCATATCCAGTGATTCT
GAAGCTTTCGTACGAGGTGACTGCCGGTCTGAGAGTTCTCGTACAGCGCTCTTAATACTT
CGCATCCGTTCAGATCCTCTCGTACTCTGGAACCTACATGCGCCTGCGTCACCTGTACGA
CAACTCGCTGGATTCTCACATCAGGAGCTCAGTGGGCCGTGGAGAGGGTTCGGGCTTCGG
TAAGACGCGTGGCTCAGGCCGAAAGGGACAAATCGCCCGTATCCTCGATACCATCGAACT
CAAGTTCGAGGGCGGTGGACTACCGCTGCCGTCACGATTACCGGCCGATGGCGGAATCGG
TCAAATCCCTCTCAGAACTGCTTACGTGTTCGTTGCTGTCGATTCCACGGATTCTGGTCC
ATACACCATCGTACACAAGGGAGTTCTCCGACATCTGCCTTCTATTGAGCAACCTTCATC
AGACGTAAAGGTACTGCCATCAACTGTACCTTCAAGAATCCGGCTTCTCACGACTTCCTC
GTATCAGTCAGAGTCTTACGGACGTCAGACAATCTGCGGAAGTCAATACACGTAAGTACC
ACACGAAGTCCCACTAAGCTTCTCTGGCTGCGAACTGTTCTTACTGCAGAGACTTCGCCA
AAGGTTCCTGCAAGCACAATCGACAAACGATCGCACTTCTTACGACCGTCGAACCAGATC
CCTTTATCACAGCCTTCCTTCTTCGACTACGGAAGGAGCGGTATATTCCAGGAACTGTTC
CTATCCACTCGCCAGAGCGAATATCTCGCAGGTATTCTCAATTCGGTTCTCGCGAGCAGA
TACTACCCGAAGCTTACTACAAGGCAGCATTC
seqHMM6001 (SEQ ID NO: 21) [Formula 5A] 
GAATGCCGTCACCTGTCAAGTCGCAGACCATCTGCACTCGGCCAGTCGGACAGTCTTTCC
GCAACCCGAATCAACCGGTCGAGCGAGGAACGTCCGATCGCAACGACAACGAATCGGCTT
CCCACCGGCACCATCCAACAGCGAGTACGTATCCGCACGAGTCCAGCTGGCCACGCAACT
ACCAGATACCTCGAGCGAGATACCCGCTCACGCGCACCGAATCAGTCTACGAAGGGAGTC
CTCGTCGCCACTCCACCTGCTGGGAAAGGCCACTCTTATGACTGTCAAGATCAAGACTGG
CGATACTGTAAACGTGATCGCAGGCAAGTCCAAGGGCCAGTTCGGCTACGTTCCGACAGT
CTCGGACTACCGTCTCAGCGTGATCGTGGAGGGCGTGTCGAGCACTAAGAAGGTACTCAA
GATTCGGACTGCGCTCAGTCTGCACGGTTCCGGTATCTTCCCAGAATCACCGATCCCTGT
CAGCAATGTGAGTACAGCAAGGAACTCCGACCTACGACTTCGGCCGGTCGGAATTCGCAT
CGGACCGGTCATCTGGAAGAGCCGGCGCCACAACTTCTACGGCTCGGCGATCTAAAGGTC
ACACGCCCTCAGAACATCGCCAGAGTTCGCTCAACCAATGATCCCACGCATTTTACACCT
GAGCTTCCCAGGAGGTACCAGGCTATCGGAGGCGTACTTCCTCCGAGGCGCCACAGTCAT
CGTAATCGCCGGTATCGTCGAGGGTTACCCGAATAGAATCCAAGGATTCCCGGGCGTCGT
TATTGGTCCTGAGAAGCATGGCGGCAAGCTACGCAGACCTGTACAGAAGGGAAGTATTGG
TTACGGCGTGGAACGCCGCTTCTTCCCTCCATTCGTTTCGATCCGACGGATTATCGTGGC
TACCAACGGCACTGTCACTCGAGCCGTCCTGCACTATCTTCGGCACGTACACGGCAAGGT
CCCTAAGATCAAGGCATCGGTCTAAACGAATCCGGTATCCCAAAGCGTCGACCTGCTCGG
ACCTTCAATGATGGCGGTCAACTGCCGCCATCGGCGCGTGCTCGCCAGAATCCCTGGCCT
AATCGCTTCATCGCTGATCTGTCCGATCGAGGACCAGTCTCAGCTACCTCTCGGTGTGCT
GCGGGTGGAGGAGTCGCGTGACTTGGTACCAGCCCAAGTCTTCGTGTACCGGCTGATCGA
CCCAGTATTCGGCACGAGCGATGTCATCGACTTGACCGTCGCCGATGGTTTCATTACCGA
ACGATTGCTTCGTCGAGGACACCTCTCCGTCGCGCCGCGGCTGACTTTCCAAAGGGACCA
ATCGCTGTCGGCGGAACCGCGTATCTTCTCTTTGATTTCGCTACTTACCGCAACCTAAGC
GTCCTATCTACAGTCCGTCGAACGCTCGACTCCCATTCCGATGCGTTCGTCGTTCTGCAT
CTCACCGCTGCCACCAGGTCTGGGCACCACCCTGGGCGAGTTACTGCGTCGTGTTCTGCT
TCACGTAATCGCAGGAACCGCGATCAGAGCGTCAGATATACACACTCGCCGGATCGAGTT
CCGACCTGGCAGTGGTGTGGGAGAGGACGTGCGCAACATCATCCGAAACCTGAAGTACCT
AGCCCTCTGTATCGCTCAGCCGTCGAGAAGGACTGTTCGCGGCCAGGATACACAACCGAA
GCCGCTCCTTCTAGCTCGGGACGAGTCACGCATACTGGTCAGTGTGGAAGTCTCCGCGCG
GACGATTCGCATTAATTCACTCGACTCCGTACACCTGCTTCCTATCATCTTGGCTGTCCC
GGGAGGACTCGGATACCGTACCCGCCAAACCGGTTTCCTCGGACAGAGTTCGACTCGGGA
CATTTCAGTCGACGCCTCGTTCTCGCCGGTCCGCATAGTGCGGTACACGGTGGTCCAACC
TGTCGTCCCTGGAAGTCTACGCCCTGATAAGCTGTCATTGCTCATCGCGACGCATACGTC
GACAGGCAACGTTCGTGCGCTGAGCCCTGCGCCAATCTGGCTCCTCTACGCTCTGAAGGC
TCAGGAGGACCGGCTACTGGTCCACTACGCATCACCGTCCAAGCACCCACCTCGCATCAC
CGTTCCTGTACGGACATTCGCAGTCATCCGACGCATCGTTACGTTGGATCTGAGCCTACG
TACTGGTAACTGCCTCCTCAACAACGTCATCGGACCTCTGGTTGAACTGGTGGGCGGTTT
ACCGGCACCGCTCACTACCCATCCTAATTTGGGCCGTAAGTCGCGTCCGGAGATTAGAAG
CGTACTCGCACCGACGACCCTGTTCCTCGGTACGAGTTAATCACCACAGGCGTCTGCCGA
GACTCTGCTCACGAACTCTACCATGTTCCCGGACTCCACGTCTGGCAGGGTCACCAATGG
CGGTGGCGGTGCTGGAACACGAGGCTGTCGGCAAGAGATATACGCGGACGCGGGCGGCCC
GACGGGCGGCACGGGCGGCCAGGGCGGACAGGTCAGAGGCTACCGGGCCAATCGTACTAT
CGCCCTCGGTCCACGAAAGCGTTCGCCGGTCACGTACGCGCTGCGAGGCACTCTCGGCAC
ACGACGCGGATCGGATGGTATCGGTTCTGGACGAACGGACATTGTCGTCCCGTCTGGTGA
GTCCGTGCGACGTACTCACCGTTCTCGTCCGTACTCGTTACCGGGCGAAAGGTACCCTAC
 [Formula 5B] 
GTACGCTTCGGGACAGGGCGGCCAGGCCGGCTTAGGCAACCAAGCGTTCACCACTGCAGT
CGAGGGAATCCCGAAGAGGTCTTCGCCTGGATCGACGATACCGCAGGAGGCCCTGATCCT
CTGCCTTTCCCGCCTTGCCGATGTGGGCCTGGTCGGCTTCGAGTCTCGGGGTAAGAGCTG
GCTGTTGACACGTCTATCACTCTGCAGCCTGGACATCTTCCGGTACGGATTCCCAACCAT
ACACCCGGGCCTGGGTATCTGCCAGCCTTCGGACGACTTACGAACGCCGCCGGCCGACAT
CCCGGGTCTTATTGACGATTCGTACCCAGGTGCTGGCCTGGGTTGCAGGTTCCTGTATCA
CATCGCACGTCGACGTTATCTGGGCAGACTGATCTCACTTCGGGCCAACACGCCTATACC
TCTTCGGCTGTCGCGAAGACCTCAGGTCACCTGCCTCTTACCTTACGCCTACAGCCTGAC
AGCTGTCCCGGAGTCTATCCTGCCAGATAAGCTGGACTCGTTCGAGGCTTCAGAGCACGA
TCCACTCGGCCTCCATATCCGACCGTCTCAACAGGGTCTCACAGCCTGGCCACAACAGGA
CCCTGCTCAAGCACTCCGTATTCCGGGCTACCCATCGCACACTGCCCACACATAAACTAT
CCCTCGCTTCTCGCAGCGCAGGACTTCAGGCGGATATATGCACTGCGTCGCGATCTTGGG
CACCACCAACCTGGGCAAGAGCACCCTGCTGAACAGGTTGTCCACCTACCATCCGTATAT
CGTCTCCCGACCTTGCTCGACAACCTATCACCAAATCGAGGGCCTGCGCGAGCTGCGTTC
GGCGCAAGAAATTTTCATTGACACGCCTGGCTTGTCGAAGCCAAAGGAGTACCGGTCTAC
ACTACTCAGGAAGCCTATCCCAGCGGCACTACACGGTGTGGACCTTATCCTGTTCGTGGT
GCAAGTTCACGGCCGTTCAGTTACGGACTTCCCGCTCCTGGCGACACTCGACCTCCATAC
AGCGGCTACTCTCCTCGTCGCAACGCTCAGTCTACCGATCATCGCGTACGAAGCGGCGTC
CACAAGGCAGCTTCCTCCACAAAGCTACCGTCCAAGCACCATCGTTGCTATCACCACTTC
GACCATCGACCGGCTATACCGCGCGAGGGTATCGAACAAGGGCCTCCTCGAAGGTTACCT
CGGTCCTGTACCGCAAGACAGTGTACGTGACGAGTCTACGGGCTTCTTCATTCTTGAGCT
GATTCGGGAAAAGATACTTGGTCGACTCACGGAGGAGATTCCATACATTGTGGGAGTCCA
GATCGCAAGGACGTACCAGTCGACTTTCACCGGCTCTCTGATCCCGTATCGCATCCCTGT
CTACCGCTGCAGTCAAAAGCTTATAATCATCGGCGTTCGTGGAGAACGAATCAAGGGCAT
AGGCGCGTCGTCACGACTTCAGCTTTCCAGTCCGGCGGACCAGCTTGTCACTCTGTCTCT
GCAAGTCAAGTAAGTCCACCTGGACTGGTCAGCAGTCTCTACGAGGAGAGCTTCGATGCG
ACCTCCGAAGAGGGTATACCCAGGTAAGACGCTGGTCCGCAAGTCGGAGGGCGACTTCCC
TTCCGATACCCACGTCCTCTTCGGCAGATATCGCCTGTACGTTCGACTGTCCGGAGCAAT
TGTCCTCATCGCCATCACCTTCCCTGGAAACGCGCTGGCTCGACCTGTAAGCTCGAGTGG
TGGCCTGTGGCTGAGAATCTGCGAGCTGGAAACTGCAAGCTCGAAGCCATCCGGACGCCG
CATGGGCCAGGGCAAGGGCCCGACAGAACGCTGGAGGCCAATCGTGGCCCAGGGCACCGT
CCTTTTCGAGCTCGCTGGCGTCCTTTCGGCCAAGCTCTACCCAGCGCTGTACGTCGCCAC
AGCAAAGTTGCCGCACTAAGTCTGCAGCTGCCAAGGCACTCTCGAGCTACGCTCTGGTTA
CATGGTCCTGTTGGTGGCGGTCAGCGGTGGTACCGACTCCGAGACACTGCTCCCTCTGCT
GGCACATACCTGGCAGAAGCACCTTATCGCCCAGCCGGCAGCGGGTGTCGATCATACACA
CCGCGTTCACTCGAGGGCACGTCCTACCTCGGTGGCTCACACGAAGCGCACCGCTCCTGA
AGCGGTATCTGCAAGCAGTCTGTCAGGTCGACACGCTGGCGGCAAGAAGGACTCCCAACA
GGAGCCGGCGGCCCGCGAGGGACGCTACCAGTCACTGCTTCGCAGGTCCAACCGCAGGAG
TTCCGCTCAAATTTTGACCGCACATCACGTCGACGACAGAATCGAAACCATACTCTTCCG
TTTGCTCCGTGGCCGACCTCATCACGGCCTGGCAGGCCTGCTCGCTGCTACGGAGAGCTC
TGCTATCGGTCAAATCTTCCGTCCGCTGCTGGTCATCTGGAAGACGGAAATCTCGTTACC
TGCGGTCGTCAAGCAGCTCTCACAGGTCTGCGACTCTACCAACGGACCTACGTACTACAA
GCGCAACGTAATCCGCTCGACGCTTCTGCCGTAATTCAGAGTCGCGTTCCTCAGGAACTC
GCGGCTGTATCCTTCAATGATTAATACGCCAATCACGGTTCGAGAGGTGTCGGGAATCGC
CACCCAGGGCTTACTTCAGTCCATCGCAGAACGTCGAGAGGCCCTGGTTCAAGCGTGCTG
 [Formula 5C] 
CAAGACACTCGATCTGGTCACCATCTCGTCAGATTCCCGAAGGCCTGTCGTTAAGATCTT
GGATTACGGCAAGTACACGTACGGTACCTCTAAGGGCTCCAAGCGGGTAAAGAAGAGTCA
AACGAGGATTACCGTCAAGGAAGTCAGCCTGATCTACGTTATTCCTACTCCGGACGGTAC
GTCTAAGACTGTCAAGCTCACGAAGTTCCTCATCGGAGGCGGTCACGTGAAGTCTCGCGT
GCTTCACCGCGGTCGCGTTCCGACGGCTTCCCAACTCACTCGAAGCGTGCTTAGGGTCGT
GCTCAGCTGGTCCGAAGTACACAAGGCGAGCTGTCGTCACTCTGCCCAGCTGGTACTGTA
CTGCTCCCGGTCTCTGCTGCAGCCGCCTCCGCGATAAACAATCTGCCGCAGTCAGGTTCA
GCGTCCTACCTTCACGTCGATGCAGGTCGCACTTGTTTCTCTTAGACAGCAGGCCTCCTA
TACCGGTTCGACGTCCATCCGCTACGACCCGATCGCCAAGGACTTCATCTGCCGACAGCG
TTTCTGCCTCAGACGAATCTTACTCAGGATCGGCCGCTACCTGCTGCGACATACGGAGCA
ATTCGTGGCGCCGCCTACTCCTGGCAACATTCCGATCCTGTTCGTCGGCACGAAGTTACG
CGTACGGAGTACTGTGCTGTTCCACGCCGCCGCACACTCGTATCGGTATGTCGTCCGACG
CTGGCTTGGTGGAGAACTCGTAAACGCCGATACGATTGCACGGATAATCCGAGCTCTTAG
CTCTCTTCCGAGACGCCCTGTACCACACGTCCTCACCCATTCAGGAAAGAAGGAGAGCTT
CTCGTACTGGGTACCGACCTCATTCCTCTCGGACGTCCTCTTCGGCATCGGTACCCTTTA
CGAGGCCCCGAGACTGCTGGGTGTGGTCGACCTCGTTCACGAACCGCTAGCGGTGAAGGA
AGCGTGTCCATTGCAGATTCCTGTGGTCGCCTGCGAGGACCGCAACTCCGCGCCGACAAG
TGTAGACCAGAACATCCCGATCAACGAGGCAGCGTTCAATTCGGCGACGCTGTCGTCCCA
CTACAGCGCACGCTTCGTCTCGAGCTCCGTCTGCACGTAAGGACACTGCGGAAAGATCGT
TCTCAGAGCGGCTTCCGGTCCGATGCTACCGCTCAGACTGCCTGCACAGGACGTCACGCG
TACAAGGCGATACAATAGCATCTATCGCCAGAGCGACGGCATCACCGATGTCTTGTCCTT
CCCATGCAGTACCGAGCGTTTCGCACCACTGGCTTCTCCAACGCAGCCTGTCGGTGACCT
CAACATCAGCCAGGTCTCAGTGGGAGTCGCAGCGTCACCTCGTACGACTCTCCGACTGCC
GGGAAGGAGTCAACTGCCTCGTCACGGCTCCCTGCACTTGCGCGGCTACGACCATCGATC
CACAACTACAGCCGCGGGAATGGTTCACACCGAGCGCATAATCCTCCTACCGCTCCCTTA
ACATCTGCAGACTCGAACCTGCTCTCATTCACGGGCTCACCGCATGCGACGCATCAACCC
TTCACGTTCCACTCGGTCGCGACGGTGGGAGGTAGTCGATGCCACCGCGATCGGACTTGG
TCGTCTCGCTGAGCAAGTCGCTGTACGCCTGGGCGGTAAGCGCAAGCACTCATATACTCC
AGTCAACGACGCTGGCGATCGAGTGATCGTGATCAACGCGTCGGGAGTCACCCTCTGTGG
CCGTAAGACCGCGTTCAAGCCTTATGACCAAGCTTCCTCTGGAACCGGTCTCAGTATTAC
GCCACGTCGTACGAGCCTGCGATCCGCAGGAATCCTATCCTACCTGTGCCAAGCGGTCCC
GGGCATGCTCCCGCCTGTCACTCTGAAGGCGCGTCCACTCGACAGCCTGCGCGTCTACCG
AGGCCGACTCCCGAGCTTCAGTGCACGGTCCCCGTATACGTACGAAGGATAACCTCTTCG
CTGCAGCGCCGGAATTTCTCGAGACAGGACTGCCATGGGCACTGGCAAGAGGTACCGCTT
CGACACCGAACGGAGCACGTTGCTGCGTGCTACAAGGCATACGCTCCTCAGTCGGTCTCC
GATTGGCACGACCGTCGCTAAGACGTCAGCCTCCGTCAGACGTGTCGAGACACTGATCAC
ACTCGCCAAGGCCCAATACCGCAGCTCCGAGCGAATTGTGCGAACCACCCTGGACACTCA
GGCAGGACAGCACTTCCTCTTCTCAGCGATTCCACTACGATACCACCCTCGCCAGGGAGG
CTACACGCGTATCCTGAAGCTGTCCGCTCGTAAGGGCGACGCTGCCCGCAAGGCCTCTTT
GGAGCTGGTCGATTAAAGCTGCGAACCGGCAAGATCTCGACCATCCCACGATCGGTTCAT
GGTACGGGACTTCCAGCGAACTCGCGTGAAGCCACTGCGCCGCAGAACCTGGGCGTCTAC
CCGGGACTGCAAGCTCGCACTGGACAGTCGCAGACTGGATTTGACCCACGCGAGTCGTCC
TCTGCGGATCGGCGGCACTCGCTCAGCGGAGAAGAAGCTCTCCGACGCTGCTGAGGAAGG
AAGCGTGAACGAGGTTCGAGAGGTTTCGGCCTATCGACTGGTGAGCATCCCGGGTACTAC
TGATTTCGTCGCACCGAGGGACCGTTTCACTGTCCTATTACTCCGAATCGCCGAGCTCCG
 [Formula 5D] 
TCGACTGGACACGTTCAGATTCCCAGCACGTCCGCTCTCCACGGATACGGGATCCATACC
TGCACACGTCGTCCTGCCGCAAACTCCAGCGGGAGTACGGGAATCCATCCGGGTCAGACG
GATCGCTGGAAGCTTACAGTACTCTCAGGCTCTCATCGTACCGTACAAGCATCTATCAAG
TCGCATCGGCGTGCTGGTCCTGCACGTCGGCTACTTACTGCTCTGGACCGCAACCTCTCT
GGCCATGCATGTGGCGGCGCTCGCTCCTCCTTCACTGCTACCGGCGGAGGTACGAACTGC
TACTGTCGCGGCAAACACGATCATCGCTTCCGCATACTGGATACGGCCTGGCGTACCGAT
CCTCATCCAGCACGTACTCGTGAGCGGCCGTCTCATCAAGCTCAAGCCTGACATTTACCT
GACGTCGCAACAGTTCGTGTTCGAATCCGGCGACTACGTCCATTCGGTCCTCTTACGCCT
ACCAAGGCACGTGCATCACTTCACTGGCCCTCTAGTGGGCGAGGGAATTGGTAAGTGTCG
CTTCACCTTCCTGGTTGAGGTCATCGGACCGAACTACGCCTAACATCACGCCCTATCCGG
ACTATCTCCGCTGCAATCCGCAGTTATGATGAAGGTCATCCTCTTCGAACTGGTCTCGCA
GCTCGGCGCTCGGGGCGACGGACCGGAGGTCAAGCTCGGCTACGCGACCAACTTCCTCAT
CGGTAAGCGTACAGCGTACGGTTACACCACGCGGTACATCGTCCGGGCGGATTACACATC
AGCGTTCGGCCCAGATCAGGACGATCCGCCTCAGAAGAGTCCGTACTGCCGCTACACAGC
AACTGGACGGCCATTCCTCGGCATCTTCTGCAAGACGCTGGACCCTGGAGGATTGTTCGG
CTCCATCCGCTCACACCGTATTAGAGATTCGGTCATTACGCCTACGCTCAAGCTGGGTAA
GGCACACATCCGACTCGTTCTCACGCGGAAGGTCCCTACGGGCGCGACACCGGTCACGCT
GTCCTTGCCTTACGAAGTATGCGCGGGACTCCGTGTGCTAGTTCGCGTCAACTAACCAAG
CCCGTCTCACAGTTCCAAGGTCTGGACAGCCACCTCAATGACGCTCCCTCCTTTGATACC
TTCCGCTGGACTCTCCTGCGTACCACGGACCGTCGGACGCGGCCCAGGCTCGGGTCAGGG
CCACACCCACGGCGAAGGACCAAAGGGATGGGAGGCCCGCAGACGTCATCCGCGAACAAT
CAGATTCGAGGGCGGCGTCACACCTCTTAGCGTTCGGCTGCCGAGGTCGGGCCCTGGATC
GGGTTCCACGCCTACTTCTGTACCGGTCGCAGTCCGTCCAGTCACGTCTGGCTACAGGTA
CAGCGCAGAGGTCTTCGCACCTCGCCTACCAACGAATCGACGCATCGAGAACATCCTGCT
CCCAGTCAAGGTGCTCACCGCCAGCTACTCGAAGGCCACTGCTGGACGGTATCCACTGGG
CACACTGGCGAATCGTCAGTCGCCGTCGAGATTCCGCTGGGCCTACTCCGACTAAACCAC
GAGCTGGACTTCACTGGAACGTACTGTCACACACCGGCCAAGCTCAAGCGCGAGCTTACG
CCTTCAATACGAGCCGGGCAGTTCACCCGACCTCATTCTGGCAACCCAATACGCCTCGCC
TCAGAGGCGTCGTATCGTCAGCAGGATTCAGGCCCAATCGAGCTGCCCACTCCGAGAGCA
CTCCTACAGAGTTCCTGGACGCTTCCTCGAGCTCACCCGGTACAACACCTCACAGAGGCG
CACTACACGACTTCGCGCCAGCACTAGCATTC
seqHMM04 (SEQ ID NO: 22)

[Formula 6A]

GAATGCGGGCCCATCGTATTTCGTTTCCGTTCTATTCGTCACAGAGCCCTGGACGATTAC
GAAGTATCCGACAGTATTCTCGAACCCTCAGCGTCTCGACAAAGTACACACACGATTCAC
ACATCGGGTACCACCTCAACAGAACTATCTCGGAGCAACAGAACCTACGGGTCTGTTTCC
GAGTTACTTATCCACGACAGCCTTACTGCGCTCAAAGTCAGGTCATCTGTATCCCTCATC
CGATTCCCAACTCGACGTATCCGAGTCGGAAACCTTCATGCTGTACAAGATCAAGTTAGG
CGACCAGGTAATCGTGATTGATGGCAAGAAGAAGGGCCTCTACGGTTCGGTGACCTTCGT
CCGATTCGCTCGGATATACGTGATCGTGGAAGGTGTCCAACATCGAAAGAAGCATCAGAA
GCCGGGAGAGTACAACTGGATTCCAGGCTCTCACTGCAGTTTCGAATCGCCTATTTCAAC
GAGCAACGTGATCACGGACTACCTTCCAATCCGTACGATCCAGTACGTCCTATCGCGTAC
ACTCTTCTTCAGCGACAAGACCCGTGTCACGGAACATCAAGGCTCAATTATTTAACTCTT
CCCGATACCTGCGACTCAACGTATTACTCTCGCAGGAATGACTCTCGAGATCCTGTACCT
GTTCGTCTACAGCCCTGCAATACGATCGCTTCAGGTTTTCGAACACGGCCAAGACGTGTC
TGTTGCTGGTCCTATTGTTGAGGGCCGTATACGGCGGACCCAGCGATTCGAGGGTTGCGT
CATCACTCATCGTCTCTTCGGCGCGACTACAAGACTTCAGGTAAGAAAGATCCACTTAGG
AACCGGAGTGGAACGTACCTTCAATCTCAGCTCAGACAAGATTTCGACAATTACTGTGGA
TCCACGAGGCATCGTGGCTCGAGCCCGTTTAGCATATCTCAGAGACAATCCAGGCAAGCT
CGCAAAGATCAAGCTATCGCAGTAACGATTACCGAATCCAGACTCGGACACATCTATCGG
CAAGTCTATGATGGCCGGACCTCTTCGGTCGTCTCGTGTGCAAGCAAACATTCCTGCAGC
CATTTCACTCGTTCTGACTCGCTTAATTGGTGATCACCCAAGACGTATCAGCAGTGTGCA
CAGAGTTGAATTCAGCGGAGATCTGCAATACGCGAGTGTCTTCGTCTTACTGCTGCGCGA
TATCGCATCAACTCAGTCTGTCGTGGGTTTCTTGCGTCGCGCACATGGATTCATTTCAGC
TTACCTTAGGAGACTGGCGCAGTTATACAGAAACCCAATCCTGCGTTTCCACGGCGACCC
GAGTTTAGAGGCAGTTCTGCGCATCCAACTGCTGATCGGTTACCTGTCAGCAACGTAAAG
AATCATACGAGGACCTCGTTTACTACATCGTTCAATTTCAATGTTATCGTTCTTCATTCA
AGGACCGTTGTCAAAGGGACTTGGTATCACTTTAGGAAAGGAACTTAGAAGAGTTTTGTT
ATACCAAATTATTGGATTCGCGATTGTATTCATCAGAATCTATACGTATATACCTGAATT
CGAATTACGATCTGGCGTCAATGAAGATGTATCTGTAATAATAGTTAATCTTAAGTTCTT
AGAATCTTACGATACTTATATACGAGAATCATCGTTCCAAAGTTCTGTTCACATATTATT
ACAGATCAAGAGTGCTTACGTTACCTACAAGAATTATATTATTGTTGAAGTTCTGGCACT
ACAAAGAGTTATCATATTATTGAGATTCCATCCAAGGTTACAAATACATCTTATTGTATT
ATCAGGATACGGATATGCTTATATCGATACAGTTATTTACAAGTTCATATCGACTCGAAT
AATCATCGTAGATGCTTTCTTCTTACCTGTAACAGTTGTTACCTATTTCGTAGAAAGATT
ATACCATCATATCGTACAAATTCAAGATTCGCTTATATTATTAATTTTCACATCGTATAT
CGAAAGACAATTCGTAGCATTATATCTGGCTATACAGTATCTAACTCTTCAATTAATCGG
ATTCACAAAGAAGCATCGTATTTCAAACACGTATTACAGATTCCTCTTACAGAATATCGA
TATCTTCGAGTTATTCCAAATCGACAGTATCATTATCTCAGTTTTAGATCTTAGCTACCG
TCGTCATAATTGTCTACAACGTATATTCATTTATATTTTATACGAATTAGTTTTATCTGG
AATATTCGAACTTCTACAAGAAAGTAATCTTGGCTACAAGAGCATTACTGAAATTATACA
ATTCTTACTATTACATACTCTCTACCTTGGAAATCTATAATTACACTGTTATTCTTTCTC
TATCAGAAGATCTTTATCTTCTATGTTCATCGATGAGTATTACCCTACTGTTTATTCAGG
ATTAGGCGGTTCTGGATATCGATCTCGTATTCTAGAACTCTATCATATATCAGGCGGACC
TCGTGGTGGTCTGGGTGGAGTTGGCGGACACGTTTTACGTATTCAAGATTTCCAAATACG
ATTCCTTAACAACGTACGTATTCATTTACACGAATATGCAGATATTGGCCAAAGTGGTAC
GATACAAATCGATATAGGTTCAAAGAATTATTCGTATTCGATATCAGTTCCTCATGGCGA
ATTCGTGAAGATATACAACAGCTTCACAGAAGAATTCCCTGTATTATCCGATATTATACA

[Formula 6B]

GATACTATCGAATCTTGGCGGTGAATCGGGTTTAGGCAATCAACGATTCCTCATATATTC
AACACGGATTCCAATCATACTACGAAACGGCATATCATATCCTGTACAGATATTATTATT
AAACTTATTCAAGTTAGCAGATGTTGGACTTGTTGGCTTCCGAATATTAGGTAAGAGTTC
ACTTTTAGTATCTATATTCAGAACACATTCGTTCATCACATCTTACGCTTTCTATACATT
CAGACCTCAGCTTGGATATACGAGCTCAGTATTATATATCAAGAAGGCTGTTGCTGATAT
TCCTGGCTTAATTAATATTACTTCAGACGGTGAAGGATTAGGTTACGAGTTCTTATACCA
TATCTATTATCGCTTCCAATTAAGACAACTTATTTTATTCAAGTTCACTCAGTCAATTCA
AACAAGTTTCGATTTCAAGTCTTATTATCCATACTTAAAGGACTACCAAGAATCTCTTGA
TTTACTATTCATCCAGGTCAAGCATTTCAAGCGAGATTTCAAGGAAATCATTCAAGTACA
GAATACGTACATATTCATACAGAAGTCTCTACGATTCAAGGGAAATTACTTACAGTCTAT
ATACGATCTATTCCATTACAAGCAAATCAAGGGATACAGTATACACATATATTAATTCGA
AATCAAGAGTTCTTATCAATATACAGTTTCGTTACAGATGTTCCTTGTTGCAATACTTGG
ATTATCTAACGACGGTAAGTCTACATTACTTAATTATTTAAAGTCAAATCAGTTCTCTAT
CTCTAGCAAGTTCCTTATCACCACCTTCGACCTTATTCACGGATACTACAGAAATTATTT
ACGACAATTCATTTTCATCGATACCCCTGGTTTATATATATCGAAGCATATCGAATACAA
GCTTTTAAACAAGTATATCTCGATAGCGTTACTGTTAGTTGATCTTATACTTTTCGTAGT
AAAGCTATTCCTGGATTTATCACAGGATTCCAAGTTATACCTGTACCTTTATTCAGAGTA
TACTTATCAGTCCTTACATTTACAAGTCAAGAAGCTTATCTATACGAACGAACTTTATCA
GTTACAGAGTATTCAATTCAAGGTCAAGTCACTTTATCGAATAGTATTCATAATTACTAT
TATACGGGTATTATCATCAGACATTATCTTCAAGGTTCATATTCAGTTAATTACTTCTAT
ACGAGAAGATCCTTTAGATTGTGTTTCTGATTCATACAAGGACTTCCTGATTATAGAATA
TATTCGTGAAAAGATTTTACGATTACGACATATCGAAATTCCTTACGAAGTTGATGTTTA
CATAGAAGATCAGAAGTACCAGAAGTTCGAGAATTATTACATTTCTTACCGTATCATTGT
ATATAGAAGAAGTCAAAAGTCCATCATCATTGGCATTACTGGACAGAAGATTAAGTACAT
TGGATCACAAGTAAGGTTCACTCTCAGTACAGAAATTTCATCACGAGTTCATCTTACATT
GTTAGTAAAGTAATTATATCCATCGAAGCATTATAGGGAACCAAGTCGGGATCTTATGCA
AGTACCGAAGAAGGTCACTCGACTCAAGACGAACATCACCAGTATATCCGGTGCTCACTG
CACCTTCGGAAAGGTGACCTTCGGTCCTTACAGGTTAAAGGCCAGTCTACAAGCTTACAT
TCAAACCTGGGAAATTTCGCCTCAAGGAAGTGCTCTTTCACGGACGGTAGGAACCACCGG
TTGGCTGTGGAATAGAATCGAATTCCAGGCTCAGCTCCGAGAAAAGCCATTCTCTCCGCG
AATGGGTGCTGGCAAGGGCAGTCTCGAACTGTGGTCAGCCAATGTGATCAGAGGCTTCGT
CCTTTTCGAGTTATATGGCGTGCGGACTCAAGAAGCGAGCTCAGCCCTATCCCAAGCAGA
AGCAAAGCTGCCGCGTTAAAGAGCGATCTCTACGTTCCAGCAACTCCTCTGCATCTGGCT
CATGGTCCTGCTCGTAGCCGTCAGTGGCGGAATTGACAGCCGTACCCTGCTGCAGACGCT
TAAGTCAGCCTTCAACAGCAGTCCTGGCTATTTCCAGGCCGCCCCGGTGGATCACGCGAG
CCGTATTCAGAGTAGTCTGGGTATCTACGGTGTCTCCGCATACGTTCTCTGGCTTTCGCC
AATACTACAGAAGTCGCAGTTCTCTGGACCAACTGTCCAGTTACGTCTCAAGCCAGGCTT
CGCATGCCCGGCCCGCCATCGACGCTACATCAGCCTGAAGTCAGCTGCCAAGGATTTCTG
TCATCTCACCATCCTGACGGCGCACCACCAGGATGATGGCATCGAAACCATTCTGCTCTC
TCTGCAGCGCGGCCTTTCAGGTATCGGCCTGCATGGACTGCAGCTATTCGAGCGATCCAA
GAAGTCGAGCAACATTCTCCGCCCTCTCCTGCCTTATCGAAAGATCGAGATTGAGTTCGA
GGACATCAAGGGTTCTCTGCAAAGTATCCGTGATAGAACTAACCGAACTGCCCATTACCA
CCGCAACGGAATCCGCCCTTCACTGTTACCGTAATTATCAGCAACCGCCATTCCTTCTCG
CTACAGGTACTCTCAAATGATCAACACGGATATCTACTACCGAGAAGTGCTATCAATTAA
CTTCAGAGGCGTTCACGTCAACATCGCCGCAGAAATCGAAGCATTGCAACTGGCAACAGC

[Formula 6C]

TTTACCTTTAGATTTGGTGAGAATCAGTAGCCTACTACGTATACCTGTTGAGAAGATCCT
GGATTACGGAAAGTACCAGTATATCTCGACTAAGCTGTCGAAGCGCAAGAAGAAGTACCA
GAATTCCATTCCGGTCAAGGAAGTATCTTTGTTCGTCAGGATCCATTTCGACGATACTGT
CGACAAGCAGATCCATCGATTCTTCTTCCTGCGCGTCGGATATCAAGTGAAGATACGGGT
TATCTTATCCGGCCGTTCGTCGTATCAACGGCAGCTGTCGATTTCTGTTTTGACGTTCGT
CCGTTACGGTTCGGAAGTCATACAATCCGACACCGTTTCGGTCTCAGGACTGTCATTCCA
TCATCGCAGAAGGCTCCTGGCTCCGACGTACGAATAAATCCTTACGGACGACACCTTCCG
TATACCACCAAGCAGTTTCATGTCGGTCGAATTGTTACTGCTCACAGAAGCGTTCCGATA
CCACGGCTCAACCAAGTTCGACGGACTACCGGCACAACATACGTTCATTAATCCTCGACG
CTCCACCAGTCTTCGAATTTGTTTAACATCTACGCTGTTCTCCCTGTCGTATTCAGTTCT
ATCCGTGAGGGAATCCCTATACGCCAAGCTACCGATTTTATTCGTTGGCACCAAGTTCAT
CGCCTGTCTCGCGGTATCATCCTCCGCATCCCAAAGATTCCCAGGATACGTACTACCGCG
CTGGCTGGGTGGCACTCTGTCAAATAGAACGACCATCCCGCTTACAATCCCACAACTATT
ACGTCTGTTCACGGATTACGCTCCTCACTCAAGACTCGCTTCACAGAAGAAGGAAATTTC
GGCCAGCTTCCGATACGGCTATCCTCTGTTCAGCATCTTGCCGGGAATTTATTCATTAGA
CACTCAGCCGCAAGAACTGCGCGTGCTTGACCAAGTCCCGGAAAAGGATGCAGTCATTGA
AGCTGGTTACCTGTTAATCCCGGTAGTTGCGAATTCCGACCACAACAAGGCGCCGACCGA
TGTAGATTGTTTCATCCCGCCAAATTATCACGCGTTCGGTCCAAGGAGTCTGAAGAAGTC
CTTCCGAACTTACGACGTTCCGGATCAAACGATTCTGTAATTACCAACCGAAATCCACCC
AATCGACCTCACGACCGCTCGGATGGTCCACCTGAAGCTGGAGCCTGGAGACTGTCCTGG
CGCGTACGCGTTAAACCCACGATACCGCGACTACGACCGAGCGACCGACGTCCTGTCGTT
CTTCGCGCAGCTGGTACCACGCATCGCACCTGCACTTCCGCAGCCACTCGGAGGCGACCT
GCGCATCAGCGCGTATCGGGTGATCTGGACGGCCTCACGGGTCCAGGCCTACCATCACCC
TCGCTCGGAGGCACTCCGATCGCATGGCAACCTCCACCTGGCCGGCTACGACCACCCGAA
GCTCACGGTTCGCGCAGCGATGCTTCTCCCTGAACAGTCAATCCTGACTCCTACGCGATA
AGCGACGAGGGTCATCCGCTATCCGAGCAGTTCGCCGCAGCTCATGAGTCGATCTGGATC
GATACACCCTGGTCGACTGCGCCAGTGGCGGATTGTGGACGCCGTCCAGGTACCGCTGGG
ACGCCTCGCCGTTCTAGTGGCCTACCGGCTCTCGGGCAAGTCCAAGGCTGGCTACTACCC
GATCGGCGACCCAGGCGACGACGTGATCGTGATCAATGCCAGTCGGGTGCAGCTGCTCGG
CGCCAAGTTCGAGTCTAAGACTTATGGTACCGGCAGCAACCGCCTCGGTCCAGCCTACGT
CGGTACCGTCAGGACGCTGAGGCGGACAGCTTACGCGCACCCTCTGCGAAGTGCCGTCAG
GGGCATGCTACCGCACCGCGATCTCCCGTCGAACTCATCCGCCAGCCTGCGAGTCTACCG
TGGCCCGAGTCGAAGCAAGGCGGCGCCGAAGCCGTCCCGCTACCATCAGTAAGTCCTCGC
CGAACGTCCTCCTGTACGGCCTATACACGCCGTCATGCGAGCGTCCAAGAGCCACCGGAC
CCATACCTCCCGCCCAACCCTGCTGCGCGCGTACGACCGACTCCTGCTGCTACTCCTCGG
CATCAGCACCACCGCTGTCAAGAGGCGAGAACCTCCGGCACCGGTGGAACGACTGATCAC
CGCCGCGAAGGTCCTGGCGGGCAGATCTGCGCGCAAGGTGTCCACAATCCTGCTCCGCGG
TTACACCCTCAGCGTACTGTTCTCGAGTATCGAGCACGGATATGGTCTCCGGGCTGGCGG
TTACACCCGGATCCTCAAGCTGGCTTCTCGCCCGGGTGACTTAGCGCTCGGTGCGCCGCT
CGAACTGGTCGATTAAGACGTCAGGAGCGAAGTCGGCTCCCAAACCGCGCCTAAGTCGAT
GTACCGAGCTTCCGGAACCTGCGAGGTAAAGCCGCTGCGCTCACACACGGGAGCCGGCTC
ACGGGACTGCAAGCGTGCGCTGAGCTCACCGTTCGGTGATCTGTCGATCGCGTGCGGCGC
ACTCCGCAGCAAGGGCGCGTTCACGGCCGTTAAGAAGAGGCGCGTCACAGCGGGAGAGGG
CAGGGTCCTCACGCGTCACTGCCCTGCGGAGGCTATTCTGGTGGCGATCGTCCAGGCGAC
CGACTTCGTAGCCACTACCGACTGGTTCCTACGCCTGCGGATCCACGCGGAATCCACCTC

[Formula 6D]

GCATCTCACTGGCAGGCCTCTCGCAGCCGCTGTACTCTCCGTATCGGGCATCAGCGCTGG
TACGGGCTGGCAACTGCGATCGCTCTCCGCCGAGCACTCAGAGCCGATCCCAGTGCGCCG
GATCGAGGCTCGCACCGTCTCGAGCATCGGCGAAATCATTCCTTACGCTCACACCCTCAG
CCGGATCGGCGTGCTGGTGTCACGGCTGGCCAGCGATACCACTGGCCTCGGAACGCGCCT
GGCGATGCATGTGGCCGCCCGTACCCCACGTCAACTGACCCTACTTCGCGTGCATCCAAT
CTGCGTGGCCTCAGGACCGCATATCCGGAGCCGATCGAAGCCTCGCCTCGCACTGCCAGC
AGGCATTGCGTACGAGCCTGTTTCTGGCCGCTTGCCGAAGCGCAACCGACGTATCAGACT
GCTGGGACAGGCGTTCGTGGTCGCGGAAACCAGGGTTGTGGGCGGTCGACTGTTCAAGCC
TCCGGCCAAGGTGCGTCTGTTCCGTCGCTGGACTGTGGGCGAGGGCATCCATAAGGCAGC
ACGCCTCTTCCCGAAGGAAGTGGCCCTGCAGGAAGGCCTATAAGCGAGACCTGCGACGTC
TTCCGCGTATCACAACCCATCGTCAATGATGAAGGTTATTCTGCTCGAGCAGGTGAAGAA
GCTGGGTACCGTCGGTGACCTACTCGAGGTTAAGCACGGCTACGCGGTTAACTTCCTGAT
TCTAAAGAGCGTAGCCTCAGATCGTACAGACTCTATAATTGCTTATTCGCCGAAGCGACA
GAGTTCAGGACTCCAACACAACGTCAACCAACTCAGGACAACGGTCAAGCCTTACAGGAA
TACCGGATTCATACCGTTATCGATTGAATCTAAGTACAATGACCGCGGTTCGCTGTTCGG
CTCGATCACTGCCAATTATATTCTCGATGTCGTTAGCTCTCCAAAGCCTTCATTGTTAAA
GTCCTGGATTGTCCTGGTCACCACTCTCGTATACTCGTCAGGCCTTACCGCTGTGACGCT
GGAACTGGAGAGTGAGGTAATAGCCAACCTGACTGTGGTCGTCACTACTGGCTAATCTTA
CCAAGCGCATTTCCGTCTCGATCTCCAACTCGAATTCATGTACCTGCCTATCTTGGAACC
AACGTCAAGCCGGATACTGTACGCTTCAGTAGTGGGTCGCGGTCGAGGCAGTGGCCTCGG
TCTCACTAATGGCACCGGACATAAGGGATCTGTAGCGCGCGATCCACGATTCTTCGTCGA
CAACTTCGAAGGCGGCTGCAGGCCTTTGTTCCATCGCCTTCCTGCTACAGGCCGTTACTC
TGGTCATTCACCAAGATTCGAGACCGTGACCGTGTGCGTACTCCTACACGCCTATATACC
TTGTACACGAGTGTGCCTGGACGAACTAACGGTTTATACGTCCATAACAACTACTGTCGA
GAGGGTTAAGGTGCTCACGGAGCTGCAACTTACGTTCCAGCATCTGCATCGAGAGAGCAT
CACTCGCGGAATCTATTCTGTCCGAAACCGGTTCAATCCAGTCTGCCACGAGTAAACACA
GTACACTCCACGTTCACTTCCAGCTTTCAACGATACCGACGCACTATTCGTCCGATTCAG
CACAACGACACTTTCACTACCGCTCAACACGTTCAGCAAACAGGGTACGCGTTACTTCAG
CCCTATACTTCACGTACTCCTCAGGAGTCCATCGAGACACTTCGTATCTACAGTATCGCC
CTTCAGAGCCCTCAGAGATTCCGAAATATCGTTACGCGATCGTTCGACGCCTCCGATCAA
TTCAGATTACTTACCGAGGTTCGGCAGCATTC

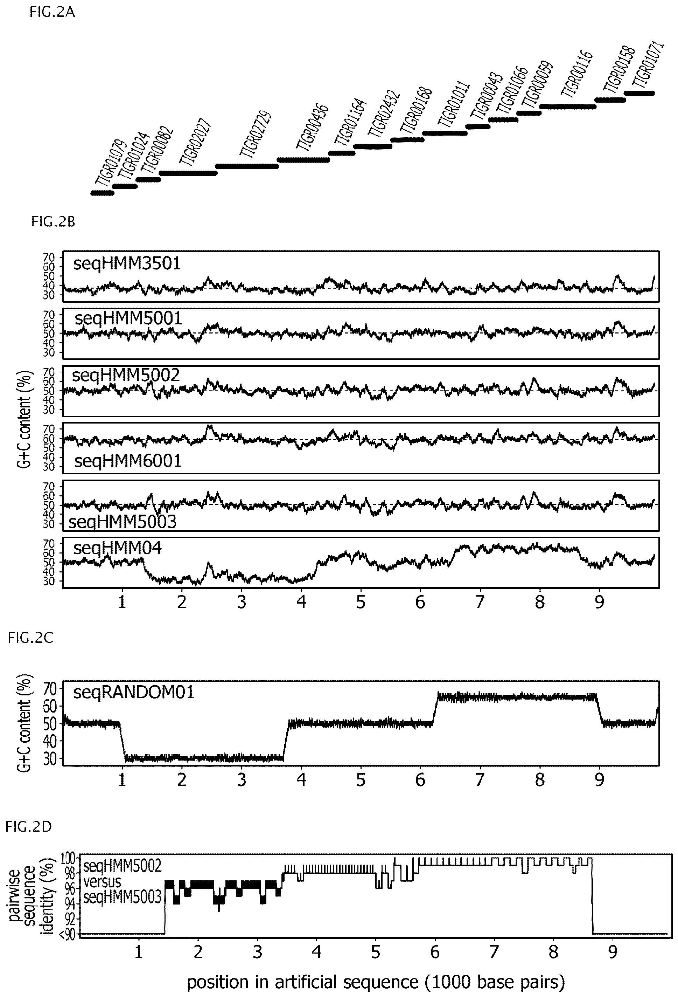

The layout of the 16 artificial CDSs in the artificial nucleic acid sequences is shown in FIG. 2A, and the GC content in each sequence is shown in FIG. 2B. The seqHMM04 was designed so that the GC content varies depending on the region. The seqHMM5002 and seqHMM5003 were designed to contain regions with varying degrees of sequence similarity to each other to mimic sequence heterogeneity between closely related species. The pairwise sequence identity of seqHMM5002 and seqHMM5003 is shown in FIG. 2D.

### (1-2) Design of an artificial nucleic acid sequence with strictly controlled GC content (SEQ ID NO: 23)

In order to accurately evaluate the effect of GC bias in the assembly, the artificial nucleic acid sequence seqRANDOM01, which is composed of a completely random sequence without artificial CDSs and has a strictly controlled GC content, was generated. The GC content of the artificial nucleic acid sequence seqRANDOM01 is shown in Figure 2C.

### seqRANDOM01 (SEQ ID NO: 23)

[Formula 7A]

GAATGCGTCACCGCTGTTATCCAAGCTCATACGTCTCTGTACAAGGCCTGGGTACTCTTC
AGCGTTCACCTGCAGTACATTTCGCCCGAGTCTTCCATACTCGATCACGAGTTCCTCTTT
CAGGGCCTCTCAACAAGATCGATTACCACTGTCCACATCCCTTTCGGGATCAAGTCAACG
AGCTACTCACTCGTTCAGAGATCACCCACAACAACCTGTACCGTCCATATCGAGCAGTAC
TCGATCCAGAAGACGCAATCTGCTCCATTTCCCATACGTCGTTCAGAGTATCCCGGATTC
TGCAGTATCAGGAATCCTCTCCGCACATTATTCGGGTACAGAGGTCTCTTACGAGAGGAC
GTCAACACAAACCCGGATTCAACGAACATCCAGAGCTTCAGGTACCAGACTACTCGGAAC
ATTCTCAGTCATCCGGGTATCGTCAAGAAGCACTGTCGAGCATCTTACAACTCGACCCTC
CGTTTCCTACATACTGCACCCAAGAGTTCTCAATCGCCTACCATACGATCTTCCCTACGA
CTGCGATCTGTCAACAGCAACCAAGCGTTCTATCGTCACGCTACCAGCATTTACCAGGTT
CCACCTGGTATCAAGAACCGGCACTATCGTTTCACGAGTACAGGGTACACTTCACGAAGC
CCTTCAGGGATATTACTCGTCGTTCAGTCTCGCACAAAGTCGGCTACTCATCAGTTACGG
AATCTGTTCGTCCCAAACCTCGCAAAGCAGTTCGGATCACATACCGATATCAGGGTCCAC
TACCTTCGAACGTCATCCTGGATCCTCAAATCGTACGCAACCCGTATCATCGTACGGAAA
GACGGAAGTACCAGTTCTCCCGTTTCTACATCTCTCGGAACAAGGTCCTGCACAACTCAG
GTACCTTATCGTTCCATCGTCATTCTGGCAGAGTCAGCAGTCCAAGCAAGACTACGTACT
CAGAAGACACAGAACGCCTGCAAACGCATTACGAGCGAACAAGAAGATCGAAATATTCTC
ATTCAGTTATATCTACTGTCTGTCTATACGTTACATTTACGAATTTCAAAGAATTCGTAC
AGTACGATATCAGAATTTACAAGCTTATTTATATCAGAGATCAAGGTACTTCAGAATTTC
ATACAAGAATATACCTGTTTACAAGTACCAGTTTATCAATCTTATCGATATTACGTCATA
TCATCTGTCAAAGAACATTCAAGATATCAAGAATCCTTTATACATCGACTATTCAAGAAG
CATCAAACAAGAATTCAAATACATACTGCCAAAGATTATCACTATCTGTTATTTCGATAT
ACAAGAGAAGAAGTCAATATACTATCGTATCAGTTTCTTATATCTCAGTCATTACAGTTT
ACGATTATATCGAACACTTATTCGTACTGTTTATCAAAGTCGATCTTATACATCAGTTTA
TACGCAATTATTCACGAAAGCATTATTATCGATCAACATTATCGAAGATTATCTGTACAC
AGATTACAAGTACATATTCCATACGTTATATCAAGATCTTCAAACGGATATTTATCGTAT
TACGATATCTGTTACTATCGATTCAAGAAGTATACAGATATTACAAACGAATTCAGAAGA
TTACGTCTTACATACTGTTTACAAACTGTATACAACGATACATTACGATCATACGAAATT
TACGTTATCTATACGATCTTTCGTATTTACGTTACTGTATCTCGTTATATTATCTTCGAG
ATATTACTTCAACAACGTTTATCGCAAATATTTACGAGTTTATATTCGCAAGGTTATCTA
TCGATTATTATTATCGTCCTTATTTCGATCAGGAATCTTTATCAAAGCATTTATCTGCTG
TTATTATCATCGTTACGTATATACAGTTTCACAAAGTCTTCGTATATATACATCGGATAT
CATTCAGAAGTATTACGTATTCGTCTTATTTATCGACAAGTCTATACAAATCAAACGTAT
ACTGTCCAATTTATTACGTACTGTTACAATACGATTTCAGACAATTCTATCAATTTCGTA
TCGTCAAATATTCAATCTGGTACAAAGTTACTGTTATTCTTCAAGACATTTACAGATTCA
AGATATCACTCTATACGAAAGAATACACCAAATCAAGAATTTCAAGCATTATTCAAGACT
TCGTATATCTGTTTCAAACATCTTACAGAAACAGTACATATTCTCAGTATATCGATCTTA
TTCAAACGATTCATACAGTCAAAGTCAAATTACGACAAATACAGTACAAAGACAAGATTT
CAACAAGCTTTACAGTATATACTTTACGAGAATCGTATTTCAGTTTCACATTACAGTTAT
TATACGATTCAGTTCATCTACGATATATCGTTCCAAATATACTTCAGTACAGTACAATTT
CTATCGTTACGAATATTCTGTACATATACGATACGTTTATTCATCACGAAGTCATATTAT
TCGTTTATTCAACGTATCTATTCGTTACTGTACAAGTTATCACAGTATTTACAATTCGAG
TATATCATCGACTCTATATTACGAATATACAAGTACGATATCAGTCATTATCCGTTTATA
TACTACGAAACAAGATATCAACTACGTTATCGTATACATACATTACGTTATACAGCTTAC
AGATCAGTTTCAGAAATATATTCGAATTTCAATACCGTACGAACTTATCAGCATTTATAT

[Formula 7B]

ATCGAATATTTCACCAAGAAGCGTATCAATACAGAAATTATCAGCATATTTCAGATCATT
TCGTTACAAGTACGTATTTACAAAGATTCAATCGAGAATTTCGTATATACGACTTCAATT
ATTTCAGTATACCATCAGTATACTTCAAGACGACTTTATTATTACACAGTTCATTCGAAA
GTATTCATTCAGGTCATTTATATATTCAGATCAGAGGTATCGTATATTCGTCAACAATAT
TATATTACGACGATCATTCAAGACTTACAGAATACTGTTTATATATCAGACAGGTTTACC
TTATCAGGAATCAAAGATTATTACTTCAGACTACGAATATACTATCCAGAGTTACATTAT
ATACATACGGTTACCTATATACGTTTATCTCTGTCATTATACAAATACGTTACAGATCTC
TATTTCAAGTATCGAACATACTTACAAGAAAGTTCACAAAGATTCTTCAATTCAAGGTAT
CGAAATACACAGATACATACGTTATATTACGTTTCCAAACTATCGATTTACACAGAAATT
CATTACTGTATCGACAAATATTTCGTACTACCAGTATTACATTTCGACATATACAGTATT
ATTCCAAGTTCAAGTATTCCAGAATCAGAAGTTCTTATTATATCGGAATTATCCAGTTTA
TCACTCGATATTACAGTTATTACCAGAATTTCAACGATATATTCGAGAGAAGTTCATATA
TTACAGTATTCGAAACCAAGATTCTTCATTTCTGCATTTATTCGATCAATATATCAGCTA
TACAAGATCTCTGTACTTATTCCACAAGATTTATATCGATTATATCTGTCGATACAACTA
TTCGAAAGAGCATATTACTTACAAAGAACTGTTCTCTATCAAAGAATACGTTCATCAATT
CAAGAAGAATCGATCAAATCTTACAGTATTACAGCTTATTACGCTATTATCGGTTTATAT
CCTATTACGAAGAATTTCTCTCGTATATCAGAAATTTACATCTGGATTATACTTCGTTAT
CACGAACTTATTCAAGTATTTCGAAATCACATACACGCCCTGCTCTATTCTCGACCGCTA
TCCTGTATATACTCACGCCAGGTACAAAGAGCCTGTCGCGTTCAAGATTCAAGTCAGGTC
ACTTCTCGACACCGACAGTCACAGATTCAACAATCACGACGTCAGACTCCGACACTGCTG
TCAAATTCGTTACCTGCTCACGGAAACTGTCGGGACTTTCGCTATTCAGTATCAGCGCCA
TCTGGAAGTCCAGGAAGTCCTTCTGTATCTTCACTGTCCTCCTCGAAACCAGCTCGTCTT
CAGTCCATTCTACACAGCTACCGATCCATACGTCTGTCCGCTGTTACAAGAGCTTCCACA
CGCATTTACCCTATACGCCTGCATACCTATCGTCACCCAGCTCAGAATTTCAACGTACGA
GGTCCTCATCTGTCCACAGACCTCGATTTACCATCAGCCTTCCAGAGTATCCCGCTCACT
TCAGGAATTCACGTACTGCATACGCCATACTGCAGCGTACGACAAGTATTCGTCAGGAGT
TCCAGTTCACCAGAACGTTCGACTACAAGGTCTCACGCACAGGAATCATACGAGGTTTCC
GCGAACTTTCTGCAAGCGTTCACAGCATATTCCACAGCCGCCAACAATACTTCCCTCAGT
TACTCGACTCATCACTGCGAGCAAGTTCTCACTGCTACTGTACGTTTCCATTCCGATCGG
TCAACCTCAGCAAGTACAACCCAAGTCTCAACGGTTTCGATCCGACACTACCGACAGATA
CAGATCCCTTATCGAACGTCAGCTGGACACAAGTCTCGTCTACTGTCGTTACAAGGACGG
AGACAGTCTTTCGAGGTACTCGCTTCGTATCTACGAAGCAGGCACCTTATCGTACTGTCT
CAGGAGTTCACTGTACGGAGTCCAGATCTTCGCAGACTATTACGCTGGTTCCACTGTCGC
TACGACAATCAAGGGTTTACAATCGCCACTTCCTCAGCCATACGAGCAACGAACTACCAT
ACTCGCGTTCAATCGCCCAATCCAGATCTCCTACAGAGATCACACGAACACTTCACCGGT
ACAGCTTACAGCCAAAGCTGTTCCAACACAGTTACACCCGGATCTCAACTTCCGAACGAA
TTCCGCACAATCAACAAGGGCGACACCTTCTGCCATTCTTCAGGCTTTCCATATCTCTGC
GCCAAACGTACGCGTATTCATACGCGATCAATACATCGCCCAGGGATACGCAGAATACTG
TTTCCCAGCAACAAGTCTCGGTCCCGAATCTGCACATTCGTATACGCTCAGTCAAGGAGG
TCGGAAAGAGTACCACACCAGTTATCAGCCGACTACTATCCGACCATACGAAGAGTTCCA
GTCCAGATCACGGTACATCTGTTCGAACCATCGGTCATCGTACGAGCTACAGCTTACACC
TCTCGAACTCTTCGTCCACAACTGTCAAGGGAAAGATACCGCAACGTTCACTCGACGAGG
AATACTTCGACAACAGGATCTGGACGAAGGGTCAACGAGAAATCAACGCAGCACTTCTCA
GGTTACAGCGTACACACACCGTTCATCTCAGAACGTCCTGTCCAAGATCTCATCATCGAC
CTCGATCACGCATCAATCCGACCAGATCTGTATACTACTGCCGAGGGTTACCGTTCAGCC

[Formula 7C]

AGGATTATCATACGTTTCTCCCGCCTCAACTCAACGGGAAACCGTCATTATCTCGATTCG
AGCCTACCTCCGATTCCAAGCCCAATCAAAGCATCCAACTGCCCAATCACCACGTCGTTC
TGCGAATTACTCGAACGACAAGGATTCGAAGGCTCAGCGATTCCGATTACAGACACTCAA
AGGCAGCTACCTTCGGACTGCTCTTTCAAGCCAGAGAATCACCACTGTACAGGCGATACA
CAGCTCAAACAGCGAAATTCCAGCGCTTATCGGAGTCAGTACGGTATTCCGGAAAGGTAC
TATCCGAAGGAGGTCTACGGTTACATCGACCATTCGTCACAGAACGTCTTACGGACTCGA
AAGCTACGAAGATCGCACAAGTCCGAAAGTCGCAGAAACTTCTCAGCCTTACGCAAGCGA
AGGGATCTTCTGCTTCAGTTCAGGTACAGGAGAGGACCTGTTTCTACCTTCCAAGATCCG
GTCTTTCCAACCTCATCCGCCATATACTGGGCATATACGACGGAATACCCGTACACGTCA
GTCAACACTCAGAAGATACCCGGAATCGTCTCGAAGAGAGCCAAACATCGTCCACATTCC
ACAACTGCAACTGGGATCCTGTACGCAAATCGCTCTACGTCTTTCACAGCCAAGGAACTC
GCTTACTACGGCAAGTTTCGGACATTCCATCGACAAAGCCCTCATTCGTCTCCGAAGATC
ACCTGTTCAAGCAATCCACAGCAAGCGATATCGGAACGGAGTTACGTCTCTCAGTACCAG
TCCAATCAGTCGAGACACTGTTATCCGGTTCGAAGACGGGAGACAAGTTTCAGGACAAGC
TACTGCGATAATCGCCACGCGTACCTGGCCTCCCAGCTGGGCCGATCTATCACGGAACGC
GCTCGAGCAGACCCGCTGGCTCGACTACAGACTGCTCGGATCGGAAGGGCCGGGTCCAGT
CCAGGTCATTACCTGGGTCTCCGGAGCTCGCTCGGCTACCACTGGACTGTCAGCACGCCT
ACGGGCATCGGCCATCAGGCTACCGCAAGCGTACGCGCCAATCCGGGATACGGGTCCGTT
ACCTCCCGTCTCAAGCGCGCTCCACCGTACCGTCGCGCAAACCAGACTCGGAAATCCCGC
GCTACCCTCCCGCTGCGACAGGTCTGTCTCTGGAATCACGGCAGGCCTGCTGCACCGGCA
CATCACGACACTCAGCTGGGCACGAGGCTGCATCCGCCCGAGACAATCGCTCGATCACTG
GGTCGCCGAACGCCGCCTGCACGATTCACATCCCAGGACTGGACTGCGGCCCGCTACTCG
CGCAATCGTACCGATCTGTCAGGGCCCTGGACGCTCCAAGGCGCTCACTTCCAACAGGCT
CACACGCCGGATCGGAGCCAGCTCGAGTCAGTACCCGAGCGACTCCCGTTCCCATCCCGC
GTTCGAGAGAGGTACACTCCCGCGGGACTCACAGCGGTCCACATCCCAGGATCTCCACCC
GAGTCCACGGAAGTCCGCGACAGTCGGGAGTCACCAAGTCCAGTCCCGCACGACCGACTC
CCTCTCACGCCAAAGGTCGACGCTGGCGACGAGTACCCGCGTACATCGGGTACCGTTCTC
CCGTCGGAGCCCTACGCGGTTTACGGTCCGTCTTCCGAGTCTCGCACGGCCGACCAGTCA
AGGTCCGAGAGCAAGCTCGGACCCTCGACTCGCGAGCCACACGACAGCTCTGCATCACGC
GACCCTCTCCACGGGCGATCTGCTCGACACGACAAAGCTGCGGCACCTCGCCTGCGAAAC
GGGCGGTTACGATCAACGGCGAGGTCAGCACCCGACCCTGCGCGAACTTTCTCTGCCGAG
CCCATCCCTCGCAAGTCCGCTGCCGGTTATCCTCTTCGACCGCACGTCGGCACTCACTGC
AGGCCGCTACACGCTTTCCTCTCCGCCAGCTGCAGCCACACCGACACTGCAGCGTACGTC
TCAGCCCGAGCCTCATCAGGACGGGTCCTCAGCGAACTACCGCGCGATCAGCTCTTCCGA
GGCCCGGATCGCGAACGTACACCCAGGCTCCACATCGAGGACGACGGCTCTCGCACCTTC
GCTGGGAGTCCATATCGGCCCACGTCGCACTTCCCTCGAAGGGCGATCCGGCATACCCGT
CGGGATTCGACCCTGTCTCCGCTCTCGAGCACGCTCACGGCATCCAGGTCAGACCCTCCT
GGCACGAAGTACGCCGGCTTCCTCACACGTCTACCGGGACCGATCGAGCATCGGCAGCCC
ACTTCCGGTCAGACTCCGCGACACCGACTCTCGGAAGCGCACTGCCAGAGGATCCCACAC
CGACGTCACCGCCTATCGCCGTACGGAGTACGGCGTACCCGTTCCACTCGCCGTCATCGG
ACGATCTCCGGTCGGAGCAAGGCCTCGTTTCCCAGGCCATTCGCCCACACGTCGGGACTT
CACCCGGTTCCACTCGCAAGGCCGTACGAGGCTGGCTCACAGCCCATACTCGAGGAGCCC
AGAGTCGGACCTCGCCGAACCACACTCGTCAGTCCGGAACCGCGACGTTCGTCGTCCACC
TGGTCAGGTACGGACTCTCGCCTCGGATCAGACGGCTCCGTACTCCTCGGATCCCGCTAC
GGCTGCCTACGTCAGACGCGTCTTCACCCGCAGGTACGTCCCGGAGCTACCTCAGGGCAC

[Formula 7D]

CAGACACTCCACGTCGCACCATCCGGAGTCTTCAGCGCGGAAGCTGGGAGAGAGGCTCCG
ACAGTCCCAACCATCCGCACAGGCTCCCTGCTTTCGACCGGAGCCCGTTCAATCCCGTCC
TCACCAGCGCAAGGCCATACCCGGTCCCAACTGGTCGACCAGCCACTGGAAGCCCACTGG
CTTCCACCCACTGCGCGTTCCTCCCACTGCTCGAGGCACAAGGTCTGCGAACGGACGCTC
GTCCGGACAGCGTCGCACTATCGAGACGCGTCTGCCGCAAGAGGCTGGTCAGGCCGTATC
AGGTCCAGCCGTTCGAGCCCAACCTACCGGGCCTCAACGCTCAACGCACCAGCTCCGCAG
TCCCATCCTCGGACTCACGAACACCGCCTGCACTGGCGCGTCATCCGTACTCGAGAGCCG
AGAGGATCGGCGATCAGCGGACGAGTCTGCTCCCAGGACCAGAGAGCTGCGAGGACTTCG
CAGGACTCGAGCGCCCTCAGATACCTGCCAGAGCCCTTCGCCTCAGTCCGGTACGGCTAC
GACTCGTCGACCGTACGGTCCCGAAGGGTCTCCGATCAGCTCCGCTGCAGGGTCAGAGAG
GTCAGCCTCACGCCTCGAATCCGGACCACCGCTCAAGCGACTCCGCATCCATCCCGCAGT
ACGCCATCCGCAGCAGTCAGCGCACTACGTTCGACGCGCTGTCGACGTCCACGCCACTAC
CGACATCGCACTCGAGCAGGTTCGCGCACGGCAGCTACTACCTGCGCTCTTCTCAGCGCG
GAGCCTGCTCCTGGATTCTCCCAGGCCTGGTACCACCTCTGCGCGCCAAGGGTATCACGG
AGGCTCTGGCTTCCGAGGGACGGGACTCTCCATCAGCGTCGCTACAGCGCATCACGCCGC
TTATACAGCACGGAGTCACCCATCAGTACACTGGGAGTATCTATTCGGAGGCTGTCTACG
CACTCGTTTCCTCAATCTGGAATCAACGACGTCGAAGCAGTTCACCTTCCTTCGTCAGAA
ATTCCACGCGAACACTACGGGTCTTACAGGTCCCACTTTCAATCCAAGGACGTATCGCTA
CGCACTCTATACGGTCCTTCGATACTCCTACTACAAGCCGAACTCACCTACCAAACCTGG
CAGTCAACATTACTCAGCCTACTGCTCTCATCCACTACGGAGATCACACTTCAGTTCCCA
AAGGCCTACATCAACTGCGAACGCCTGTCAAGTATCCACTCTACGATCTCGCTATCGGAC
TTACGAGACCCATTCGGTTTCAACAGAGTCTGCTCCGATTCGACAGTTCCATATCCGAGA
ATCTGGCCTTCGGTTCTCAGTCAGTCAAAGTACTCAGCCGTACAGATCAGACGAGCGATC
ACTCATACGGTTTCACGAGAAACCGACTACGAGAGTCAGCTATCTGCTGCAAAGTCGAGT
TTCACAGGCCCAACCAGATCGAAATTCGCAAACGGTCCAATCAGAACGGCGACTCTACAT
CCATTCTGGACTACGAGGTATCAAGCCCTCGGATCAACTGTCAAACCTCGACAACTCAGA
CTTCGGGACTACGTCGAAATCACAGACATCTCCGCAAACGTTACGGCAACCAAGCCTTAC
TACCAGCTTCTCACTGGTATCTGGGAAAGACCTGGCTATCGATCCAACCTCATCGTTCAG
TACCTCAAACGAGACGGTACCGAAGTTACGTTCTGCAGTTCACGTTCCAAGTCCTCGATT
CGCACCATCAGTCACGATATACCCACAGGTTCGTACGTACGAGTTCCCAGTACCTATACA
GAGGTCGTTCGCCTATATCTGGCACTCGAAGAGTCTTACACCTCGGAAGCAGATCTCTAC
CAACACGTTCACCGTTATCGGAGGTCAGCGTAGCATTC

All the artificial nucleic acid sequences of SEQ ID NOs: 17 to 23 were confirmed to have negligible similarity to sequences registered in public databases such as NCBI (no sequences showing a similarity with an expectation value (E-value) of 0.1 or more by BLAST were detected).

Artificial nucleic acids consisting of the sequences of SEQ ID NOs: 17 to 23 were chemically synthesized by entrusting the work to GenScript Japan Co., Ltd. The artificial nucleic acids were inserted into a plasmid vector (pUC57), and the plasmid was amplified and purified by standard procedures. The restriction enzyme sites introduced at the ends of the artificial nucleic acid sequences were cleaved, and the artificial nucleic acids were separated by agarose gel electrophoresis and purified.

### 2. Assembly performance of artificial nucleic acids (1)

Using the TruSeq DNA Nano Kit (Illumina), sequence libraries were individually prepared for each of the artificial nucleic acids consisting of sequences of SEQ ID NOs: 17 to 23, and sequencing was performed using the MiSeq system (Illumina) (2×251 bp sequencing reads). After quality control using fastp (Chen, et al., Bioinformatics, 2018,34: i884-i890), sequencing reads were randomly sampled to vary coverage, and assembled using the default settings in two assemblers: MEGAHIT (Li, et al., Bioinformatics, 2015,31:1674-1676) and SPAdes (Bankevich, et al., J. Comput. Biol., 2012,19:455-477).

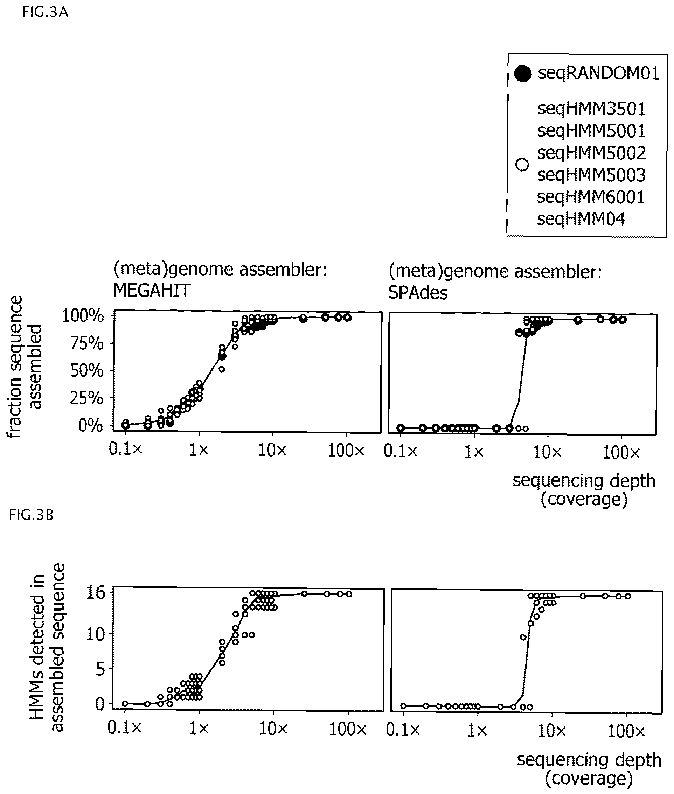

The percentage of artificial nucleic acid sequences recovered by assembly is shown in Figure 3A. The results of both MEGAHIT (left) and SPAdes (right) indicated a sigmoidal relationship between coverage depth and assembly completeness, and also showed that complete assembly was achieved even with minimal coverage (10×).

The number of marker genes in the artificial nucleic acid sequences recovered by assembly, detected by QUAST (Gurevich, et al., Bioinformatics, 2013, 29:1072-1075) and CheckM, is shown in FIG. 3B. Note that seqRANDOM01 was not included in the CheckM analysis. All the 16 genes were detected even at minimum coverage (10×), further indicating that complete assembly was achieved.

These results confirmed that the artificial nucleic acids consisting of SEQ ID NOs: 17-23 are useful for evaluating assembly completeness.

### 3. Assembly performance of artificial nucleic acids (2)

A sequencing library was prepared for an equimolar mixture of the artificial nucleic acids consisting of sequences of SEQ ID NOs: 17 to 23 using the DNA Prep kit (Illumina), and sequencing was performed using the NextSeq system (Illumina) (2×151 bp sequencing reads). Following quality control using fastp and sampling of the sequencing reads, the reads were assembled using the default settings of SPAdes.

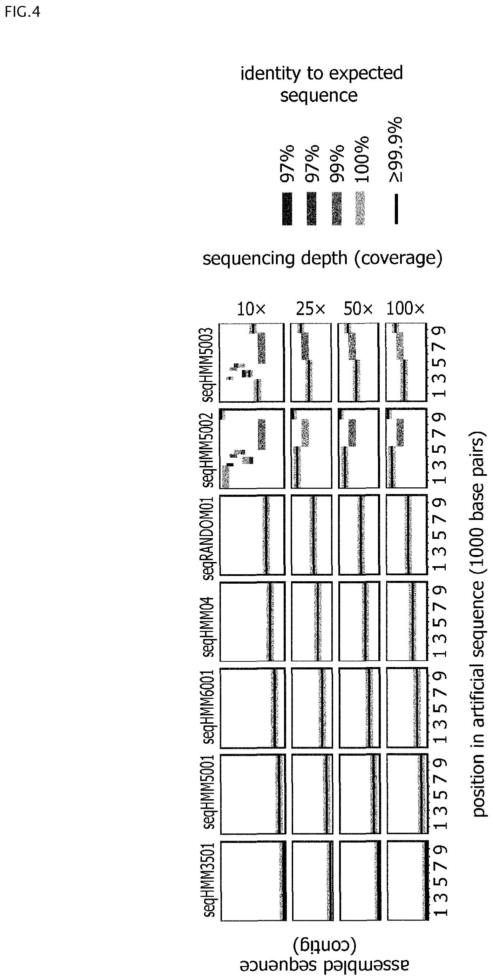

The results are shown in FIG. 4. In the figure, the shade of gray represents the sequence identity between the assembled artificial sequence and the predicted artificial sequence, and the region with 99.9% or higher identity is highlighted by a solid black line. seqHMM3501, seqHMM5001, seqHMM6001, seqHMM04 and seqRANDOM01, of which the sequences are not similar to each other, were all assembled as a single contig. These results indicated that these sequences are suitable for evaluating assembly performance. On the other hand, seqHMM5002 and seqHMM5003 were co-assembled due to high sequence similarity, resulting in fragmented assemblies. These results indicated that seqHMM5002 and seqHMM5003 are useful for evaluating sequence similarity for assembly performance.

### 4. Evaluation of GC bias by artificial nucleic acids

Sequence libraries were prepared and sequenced for each of the artificial nucleic acids seqHMM04 (SEQ ID NO: 22) and seqRANDOM01 (SEQ ID NO: 23), which were designed so that the GC content varies depending on the region. Based on the sequencing reads, coverage was calculated using BBMap (https://www.osti.gov/biblio/1241166).

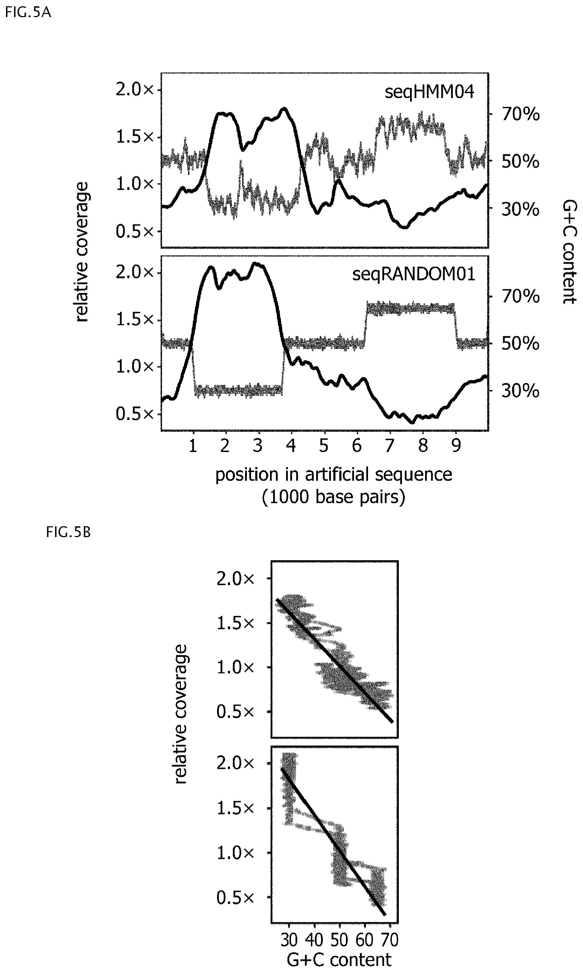

FIG. 5A shows plots of relative coverage (black line) and GC content (gray line) along the positions in seqHMM04 and seqRANDOM01. Also, FIG. 5B shows a scatter plot of the relative coverage and GC content in FIG. 5A. It was found that there was a strong correlation between sequencing coverage and GC content, and coverage of high GC content regions was underestimated. These results demonstrate that seqHMM04 and seqRANDOM01 are useful for evaluating GC bias.

### 5. Quantitative performance of artificial nucleic acids

Two types of mixtures containing artificial nucleic acids consisting of the sequences of SEQ ID NOs: 17 to 23 in different ratios were prepared. Sequence libraries were prepared, sequencing was performed, and sequencing reads were sampled by the same procedures as in 3 above.

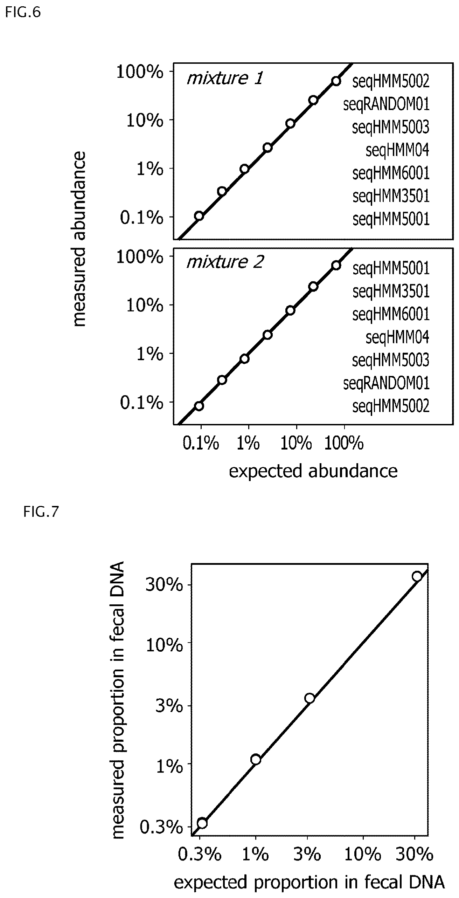

The results are shown in FIG. 6. The X-axis indicates the estimated abundance (relative value) of each artificial nucleic acid, and the Y-axis indicates the measured abundance (relative value) of each artificial nucleic acid. Quantification of the number of reads from artificial nucleic acids showed excellent agreement between estimated and measured abundances in all the mixtures.

Next, an equimolar mixture of the artificial nucleic acids consisting of sequences of SEQ ID NOs: 17 to 23 was added to a human fecal microbiota DNA sample at different mass ratios (0.3%, 1%, 3%, and 31%), and a sequence library was prepared, sequencing was performed, and sequencing reads were sampled by the same procedures as in 3 above. Human fecal microbiota DNA was prepared from human feces using the ISOSPIN Fecal DNA Kit (Nippon Gene Co., Ltd.) with reference to a previously published article (Tourlousse, et al., Microbiome, 2021,9:95).

The results are shown in FIG. 7. The X-axis indicates the estimated relative ratio of the artificial nucleic acid to the human fecal microbiota DNA based on the concentration calculation, and the Y-axis shows the relative ratio of the artificial nucleic acid to the human fecal microbiota DNA based on the actual measurement value. The relative ratio based on the actual measurement value was consistent with the estimated value based on the calculation. These results indicated that the artificial nucleic acids consisting of the sequences of SEQ ID NOs: 17 to 23 can be used as reliable internal standards for precise absolute quantification of the amount of microorganisms.

## Claims

1. A nucleic acid molecule comprising an artificial nucleic acid sequence and/or a complementary sequence thereof, or a partial fragment sequence thereof, the artificial nucleic acid sequence consisting of:
(1) one copy each of artificial genes encoding the following non-naturally occurring sequences (a) to (p):
(a) an amino acid sequence of SEQ ID NO: 1 and a stop codon;
(b) an amino acid sequence of SEQ ID NO: 2 and a stop codon;
(c) an amino acid sequence of SEQ ID NO: 3 and a stop codon;
(d) an amino acid sequence of SEQ ID NO: 4 and a stop codon;
(e) an amino acid sequence of SEQ ID NO: 5 and a stop codon;
(f) an amino acid sequence of SEQ ID NO: 6 and a stop codon;
(g) an amino acid sequence of SEQ ID NO: 7 and a stop codon;
(h) an amino acid sequence of SEQ ID NO: 8 and a stop codon;
(i) an amino acid sequence of SEQ ID NO: 9 and a stop codon;
(j) an amino acid sequence of SEQ ID NO: 10 and a stop codon;
(k) an amino acid sequence of SEQ ID NO: 11 and a stop codon;
(l) an amino acid sequence of SEQ ID NO: 12 and a stop codon;
(m) an amino acid sequence of SEQ ID NO: 13 and a stop codon;
(n) an amino acid sequence of SEQ ID NO: 14 and a stop codon;
(o) an amino acid sequence of SEQ ID NO: 15 and a stop codon; and
(p) an amino acid sequence of SEQ ID NO: 16 and a stop codon;
(2) artificial intergenic sequences for linking the artificial genes, each independently consisting of a non-naturally occurring random sequence of 10 to 60 nucleotides in length; and
(3) a front spacer sequence and an end spacer sequence, each independently consisting of a non-naturally occurring random sequence of 200 to 400 nucleotides in length.

2. The nucleic acid molecule according to claim 1, wherein the GC content of the artificial nucleic acid sequence is 30 to 60%.

3. The nucleic acid molecule according to claim 1, wherein the artificial nucleic acid sequence is selected from the group consisting of SEQ ID NOs: 17 to 22.

4. The nucleic acid molecule according to any one of claims 1 to 3, wherein the partial fragment sequence is at least 300 nucleotides in length.

5. A nucleic acid molecule comprising an artificial nucleic acid sequence of SEQ ID NO: 23 and/or the complementary sequence thereof, or the partial fragment sequence thereof.

6. The nucleic acid molecule according to claim 5, wherein the partial fragment sequence is at least 300 nucleotides in length.
